# DEMANDE DE BREVET EUROPEEN

(11) **EP 1 570 834 A1**
(43) Date de publication de la demande: **07.09.2005**
(21) Numéro de dépôt: 05300161.6
(22) Date de dépôt: 02.03.2005
(51) Int. Cl.: A61K 7/09, C07C 323/58

(54) **Composition de deformation permanente des cheveux contenant au moins un dicarboxydithiol**

(30) Priorité: 02.03.2004 FR 0450417
(71) Demandeur: L'OREAL, 75008 Paris (FR)
(72) Inventeur: Malle, Gérard, 77580 Villiers Sur Morin (FR); Blaise, Christian, 94160 Saint Mande (FR)
(74) Mandataire: Michelet, Alain

(57) **Abrégé**

L'invention concerne la composition aqueuse réductrice, pour le premier temps d'une opération de déformation permanente des cheveux, contenant, dans un milieu cosmétiquement acceptable, en tant qu'agent réducteur, un ou plusieurs dicarboxydithiols de formule générale (I) et/ou de formule générale (II) : 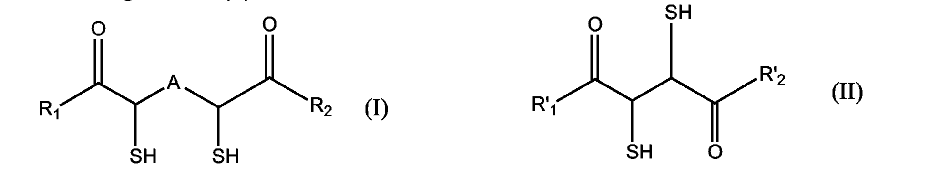 dans lesquelles :
R₁, R₂, R'₁, R'₂ représentent indépendamment OR₃ ou NR₄R₅, où R₃ est H ou un groupe alkyle linéaire ou ramifié en C₁-C₅ éventuellement substitué par un ou deux radicaux OR₇, où R₇ est H ou un radical alcoxy en C₁-C₃,
R₄, R₅ sont indépendamment H ou un groupe alkyle linéaire ou ramifié en C₁-C₅ éventuellement substitué par 1 ou 2 radicaux choisis parmi les radicaux hydroxy, méthoxy, éthoxy, méthylamino, éthylamino et diméthylamino,
R₁ et R₂ ne représentant pas simultanément un radical hydroxyle et R'₁ et R'₂ ne présentant pas simultanément un radical hydroxyle ;
A représente soit (CH₂)ₙ où n est un entier variant de 1 à 4, soit un radical - (CH₂)ₘ-X-(CH₂)ₚ- dans lequel m est égal à 1 ou 2, p est égal à 1 ou 2 et X représente O, S, NHCO ou NR₆, où R₆ représente H ou CH₃, et/ou un ou plusieurs de leurs sels organiques et minéraux.

## Description

La présente invention a pour objet une composition cosmétique réductrice, pour le premier temps d'une opération de déformation permanente des cheveux, contenant, en tant qu'agent réducteur, au moins un dicarboxydithiol. Elle vise également un procédé de déformation permanente des cheveux mettant en oeuvre ces dicarboxydithiols.

La technique la plus usuelle pour obtenir une déformation permanente des cheveux consiste, dans un premier temps à réaliser l'ouverture des liaisons disulfures de la kératine (cystine) à l'aide d'une composition contenant un agent réducteur, puis après avoir de préférence rincé les cheveux, à reconstituer dans un second temps les dites liaisons disulfures en appliquant sur les cheveux préalablement mis sous tension par des bigoudis ou autres ou mis en forme ou lissés par d'autres moyens, une composition oxydante encore appelée fixateur de façon à donner à la chevelure la forme recherchée. Cette technique permet indifféremment de réaliser soit l'ondulation des cheveux, soit leur défrisage, leur crêpage ou leur lissage.

Les compositions réductrices généralement utilisées pour la première étape d'une opération de permanente contiennent à titre d'agent réducteur des sulfites, des bisulfites, ou de préférence des thiols. Parmi ces derniers, ceux couramment utilisés sont l'acide thioglycolique, la cystéamine, l'acide thiolactique, la cystéine et le monothioglycolate de glycérol. L'acide thioglycolique est particulièrement efficace pour réduire les liaisons disulfures de la kératine à pH alcalin, notamment sous forme de thioglycolate d'ammonium, et constitue le produit le plus utilisé en permanente. On a toutefois constaté que l'acide thioglycolique devait être utilisé en milieu suffisamment basique (en pratique à pH supérieur ou égal à 8,5) si on voulait obtenir une frisure satisfaisante en intensité. Outre l'inconvénient de dégager une odeur désagréable nécessitant l'usage de parfums plus ou moins efficaces pour masquer les odeurs, la combinaison acide thioglycolique - pH alcalin conduit à des dégradations de la fibre capillaire.

Les sulfites ou bisulfites ont été utilisés antérieurement aux thiols en général et à l'acide thioglycolique en particulier. Contrairement aux thiols, ils sont utilisés à un pH acide généralement compris entre 4 et 6. Cependant, le degré de frisure obtenu est très inférieur et loin d'être satisfaisant.

La cystéine produit une odeur beaucoup plus faible que celle de l'acide thioglycolique mais le degré de frisure obtenu est également très inférieur et loin d'être satisfaisant. De plus, la cystéine nécessite l'utilisation d'un pH très alcalin.

Le monothioglycolate de glycérol est également très malodorant. Il est, par contre, utilisé à un pH proche de la neutralité, mais ses performances sont notablement inférieures à celles de l'acide thioglycolique.

La cystéamine peut être utilisée sur une plus large gamme de pH. Son efficacité est voisine de celle de l'acide thioglycolique mais elle conduit également à des dégradations importantes de la fibre capillaire.

Diverses études ont été conduites en vue de remédier aux inconvénients de ces agents réducteurs, et à cet effet, il a été proposé l'emploi de nouveaux composés ou systèmes réducteurs. Cependant, très peu de dithiols ont été proposés. Dans le passé, 2 dithiols avaient notamment été largement étudiés : le dithiothréitol DTT et l'acide 2,5-dimercaptoadipique mais ils n'ont jamais été développé pour la déformation permanente des cheveux, en particulier à cause d'une odeur épouvantable pour le DTT et d'une activité insuffisante pour l'acide dimercaptoadipique. De façon plus récente, dans la demande de brevet EP-A-0721772, il a déjà été proposé d'utiliser l'acide 2,3-dimercaptosuccinique qui s'est avéré moins efficace que l'acide thioglycolique. Il a également été proposé dans le brevet US 5350572 d'utiliser des polyoxyéthylèneglycols dimercaptoalkyesters. Si ces composés possèdent une certaine efficacité, leur conservation dans le temps n'est pas satisfaisante.

Après diverses études, la demanderesse a maintenant découvert de façon tout à fait surprenante et inattendue une nouvelle famille de dicarboxydithiols qui permet d'obtenir une composition réductrice conduisant à une frisure satisfaisante en intensité et en tenue et qui conduit également à une dégradation moins importante du cheveu par rapport aux compositions précédentes.

La présente invention a donc pour objet une composition aqueuse réductrice, pour le premier temps d'une opération de déformation permanente des cheveux, contenant, dans un milieu cosmétiquement acceptable, en tant qu'agent réducteur, un ou plusieurs dicarboxydithiols de formule générale (1) et/ou de formule générale (11) suivantes : 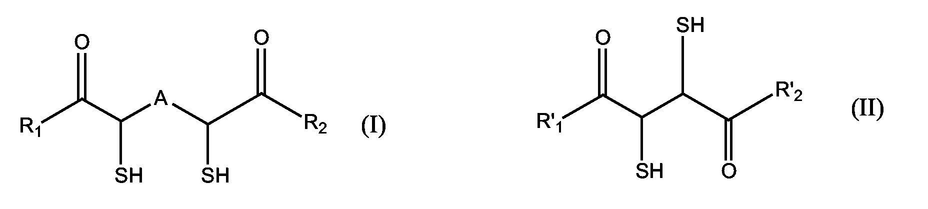 dans lesquelles :
R₁, R₂, R'₁, R'₂ représentent indépendamment OR₃ ou NR₄R₅, où R₃ est H ou un groupe alkyle linéaire ou ramifié en C₁-C₅ éventuellement substitué par un ou deux radicaux OR₇, où R₇ est H ou un radical alcoxy en C₁-C₃,
R₄, R₅ sont indépendamment H ou un groupe alkyle linéaire ou ramifié en C₁-C₅ éventuellement substitué par 1 ou 2 radicaux choisis parmi les radicaux hydroxy, méthoxy, éthoxy, méthylamino, éthylamino et diméthylamino,
R₁ et R₂ ne représentant pas simultanément un radical hydroxyle et R'₁ et R'₂ ne représentant pas simultanément un radical hydroxyle ;
A représente soit (CH₂)ₙ où n est un entier variant de 1 à 4, soit un radical - (CH₂)ₘ-X-(CH₂)p- dans lequel m est égal à 1 ou 2, p est égal à 1 ou 2 et X représente O, S, NHCO ou NR₆, où R₆ représente H ou CH₃,
et/ou un ou plusieurs de leurs sels organiques et minéraux.

On entend par déformation permanente, le frisage permanent (permanente), le défrisage ou le décrêpage des cheveux.

Parmi les composés de formule générale (1) et (11), on peut notamment citer les composés préférés ci-après :
- l'acide 2,3-dimercapto-4-méthylamino-4-oxo-butanoïque,
- l'acide 2,3-dimercapto-4-éthylamino-4-oxo-butanoïque,
- l'acide 2,3-dimercapto-4-propylamino-4-oxo-butanoïque,
- l'acide 2,3-dimercapto-4-butylamino-4-oxo-butanoïque,
- l'acide 2,3-dimercapto-4-[(1-méthyléthyl)amino]-4-oxo-butanoïque,
- l'acide 2,3-dimercapto-4-[(2-méthylpropyl)amino]-4-oxo-butanoïque,
- l'acide 2,3-dimercapto-4-[(3-méthylbutyl)amino]-4-oxo-butanoïque,
- l'acide 2,3-dimercapto-4-[(2-hydroxyéthyl)amino]-4-oxo-butanoïque,
- l'acide 2,3-dimercapto-4-[(2-hydroxypropyl)amino]-4-oxo-butanoïque,
- l'acide 2,3-dimercapto-4-[(2-diméthylaminoéthyl)amino]-4-oxo-butanoïque,
- l'acide 2,3-dimercapto-4-[(3-diméthylaminopropyl)amino]-4-oxo-butanoïque,
- l'acide 2,3-dimercapto-butanedioïque, monométhyl ester,
- l'acide 2,3-dimercapto-butanedioïque, monoéthyl ester,
- l'acide 2,3-dimercapto-butanedioïque, monopropyl ester,
- l'acide 2,3-dimercapto-butanedioïque,mono(1-méthyléthyl)ester,
- l'acide 2,3-dimercapto-butanedioïque, monobutyl ester,
- l'acide 2,3-dimercapto-butanedioïque, mono(2-méthylpropyl)ester,
- l'acide 2,3-dimercapto-butanedioïque, monopentyl ester,
- l'acide 2,3-dimercapto-butanedioïque, mono(3-méthylbutyl)ester,
- le 2,3dimercapto-butanediamide,
- le 2,3dimercapto-N,N'-diméthyl-butanediamide,
- le 2,3dimercapto-N,N'-diéthyl-butanediamide,
- le 2,3dimercapto-N,N'-bis(2-hydroxyéthyl)-butanediamide,
- le 2,3dimercapto-N,N'-bis(2-hydroxypropyl)-butanediamide,
- l'acide 2,3-dimercapto-butanedioïque, diméthyl ester,
- l'acide 2,3-dimercapto-butanedioïque, diéthyl ester,
- l'acide 2,3-dimercapto-butanedioïque, dipropyl ester,
- l'acide 2,3-dimercapto-butanedioïque, bis(1-méthyléthyl)ester,
- l'acide 2,3-dimercapto-butanedioïque, dibutyl ester,
- l'acide 2,3-dimercapto-butanedioïque, bis(2-méthylpropyl)ester,
- l'acide 2,3-dimercapto-butanedioïque, bis(1,1-diméthyléthyl)ester,
- l'acide 2,3-dimercapto-butanedioïque, bis(1-méthylpropyl)ester,
- l'acide 2,3-dimercapto-butanedioïque, bis(3-méthylbutyl)ester,
- l'acide 2,3-dimercapto-butanedioïque, bis(1-ethylpropyl)ester,
- l'acide 2,3-dimercapto-butanedioïque, bis(2-méthoxyéthyl) ester,
- l'acide 2,3-dimercapto-butanedioïque, bis(2-éthoxyéthyl) ester,
- l'acide 2,4-dimercapto-5-méthylamino-5-oxo-pentanoïque,
- l'acide 2,4-dimercapto-5-éthylamino-5-oxo-pentanoïque,
- l'acide 2,4-dimercapto-5-propylamino-5-oxo-pentanoïque,
- l'acide 2,4-dimercapto-5-butylamino-5-oxo-pentanoïque,
- l'acide 2,4-dimercapto-5-[(1-méthyléthyl)amino]-5-oxo-pentanoïque,
- l'acide 2,4-dimercapto-5-[(2-méthylpropyl)amino]-5-oxo-pentanoïque,
- l'acide 2,4-dimercapto-5-[(3-méthylbutyl)amino]-5-oxo-pentanoïque,
- l'acide 2,4-dimercapto-5-[(2-hydroxyéthyl)amino]-5-oxo-pentanoïque,
- l'acide 2,4-dimercapto-5-[(2-hydroxypropyl)amino]-5-oxo-pentanoïque,
- l'acide 2,4-dimercapto-5-[(2-diméthylaminoéthyl)amino]-5-oxo-pentanoïque,
- l'acide 2,4-dimercapto-5-[(3-diméthylaminopropyl)amino]-5-oxo-pentanoïque,
- l'acide 2,4-dimercapto-pentanedioïque, monométhyl ester,
- l'acide 2,4-dimercapto-pentanedioïque, monoéthyl ester,
- l'acide 2,4-dimercapto-pentanedioïque, monopropyl ester,
- l'acide 2,4-dimercapto-pentanedioïque, mono(1-méthyléthyl)ester,
- le 2,4-dimercapto-pentanediamide,
- le 2,4-dimercapto-N,N'-diméthyl-pentanediamide,
- le 2,4-dimercapto-N,N'-diéthyl-pentanediamide,
- le 2,4-dimercapto-N,N'-bis(2-hydroxyéthyl)-pentanediamide,
- le 2,4-dimercapto-N,N'-bis (2-hydroxypropyl)-pentanediamide,
- l'acide 2,4-dimercapto-pentanedioïque, diméthyl ester,
- l'acide 2,4-dimercapto-pentanedioïque, diéthyl ester,
- l'acide 2,4-dimercapto-pentanedioïque, dipropyl ester,
- l'acide 2,4-dimercapto-pentanedioïque, dibutyl ester,
- l'acide 5-carbamoyl-2,5-dimercapto-valérique,
- l'acide 2,5-dimercapto-6-méthylamino-6-oxo-hexanoïque,
- l'acide 2,5-dimercapto-6-éthylamino-6-oxo-hexanoïque,
- l'acide 2,5-dimercapto-6-propylamino-6-oxo-hexanoïque,
- l'acide 2,5-dimercapto-6-[(2-hydroxyéthyl)amino]-6-oxo-hexanoïque,
- l'acide 2, 5 - dimercapto - 6 -[(2-hydroxypropyl)amino]- 6 - oxo - hexanoïque,
- l'acide 2,5-dimercapto-6-[(2-diméthylaminoéthyl)amino]-6-oxo-hexanoïque,
- l'acide 2,5-dimercapto-6-[(3-diméthylaminopropyl)amino]-6-oxo-hexanoïque,
- l'acide 2,5-dimercapto-hexanedioïque, monométhyl ester,
- l'acide 2,5-dimercapto-hexanedioïque, monoéthyl ester,
- l'acide 2,5-dimercapto-hexanedioïque, monopropyl ester,
- le 2,5-dimercapto-hexanediamide,
- le 2,5-dimercapto-N,N'-diméthyl-hexanediamide,
- le 2,5-dimercapto-N,N'-diéthyl-hexanediamide,
- le 2,5-dimercapto-N,N'-bis(2-hydroxyéthyl)-hexanediamide,
- le 2,5-dimercapto-N,N'-bis(2-hydroxypropyl)-hexanediamide,
- le 2,5-dimercapto-N,N,N',N'-tétraméthyl-hexanediamide,
- l'acide 2,5-dimercapto-hexanedioïque, diméthylester,
- l'acide 2,5-dimercapto-hexanedioïque, diéthylester,
- l'acide 2,5-dimercapto-hexanedioïque, dipropylester,
- l'acide 2,6-dimercapto-7-méthylamino-7-oxo-heptanedioïque,
- l'acide 2,6-dimercapto-7-éthylamino-7-oxo-heptanedioïque,
- l'acide 2,6-dimercapto-7-propylamino-7-oxo-heptanedioïque,
- l'acide 2,6-dimercapto-7-[(2-hydroxyéthyl)amino]-7-oxo-heptanedioïque,
- l'acide 2,6-dimercapto-7-[(2-hydroxypropyl)amino]-7-oxo-heptanedioïque,
- l'acide 2,6-dimercapto-7-[(2-diméthylaminoéthyl)amino]-7-oxo-heptanedioïque,
- l'acide 2,6-dimercapto-7-[(3-diméthylaminopropyl)amino]-7-oxo-heptanedioïque,
- l'acide 2,6-dimercapto-heptanedioïque, monométhyl ester,
- l'acide 2,6-dimercapto-heptanedioïque, monoéthyl ester,
- l'acide 2,6-dimercapto-heptanedioïque, monopropyl ester,
- le 2,6-dimercapto-heptanediamide,
- le 2,6-dimercapto-N,N'-diméthyl-heptanediamide,
- le 2,6-dimercapto-N,N'-bis(2-hydroxyéthyl)-heptanediamide,
- le 2,6-dimercapto-N,N'-bis(2-hydroxypropyl)-heptanediamide,
- l'acide 2,6-dimercapto-heptanedioïque, diméthylester,
- l'acide 2,6-dimercapto-heptanedioïque, diéthylester,
- l'acide 2,6-dimercapto-heptanedioïque, dipropylester,
- l'acide 2,7-dimercapto-8-méthylamino-8-oxo-octanedioïque,
- l'acide 2,7-dimercapto-8-éthylamino-8-oxo-octanedioïque,
- l'acide 2,7-dimercapto-8-propylamino-8-oxo-octanedioïque,
- l'acide 2,7-dimercapto-8-[(2-hydroxyéthyl)amino]-8-oxo-octanedioïque,
- l'acide 2,7-dimercapto-8-[(2-hydroxypropyl)amino]-8-oxo-octanedioïque,
- l'acide 2,7-dimercapto-8-[(2-diméthylaminoéthyl)amino]-8-oxo-octanedioïque,
- l'acide 2,7-dimercapto-8-[(3-diméthylaminopropyl)amino]-8-oxo-octanedioïque,
- l'acide 2,8-dimercapto-octanedioïque, monométhyl ester,
- l'acide 2,8-dimercapto-octanedioïque, monoéthyl ester,
- l'acide 2,8-dimercapto-octanedioïque, monopropyl ester,
- le 2,8-dimercapto-heptanediamide,
- le 2,8-dimercapto-N,N'-diméthyl-heptanediamide,
- le 2,8-dimercapto-N,N'-bis (2-hydroxyéthyl)-heptanediamide,
- le 2,8-dimercapto-N,N'-bis (2-hydroxypropyl)-heptanediamide,
- l'acide 2,8-dimercapto-heptanedioïque, diméthylester,
- l'acide 2,8-dimercapto-heptanedioïque, diéthylester,
- l'acide 2,8-dimercapto-heptanedioïque, dipropylester,
- l'acide 2,4-dimercapto-3-oxa-5-méthylamino-5-oxo-pentanoïque,
- l'acide 2,4-dimercapto-3-oxa-5-éthylamino-5-oxo-pentanoïque,
- l'acide 2,4-dimercapto-3-oxa-5-propylamino-5-oxo-pentanoïque,
- l'acide 2,4-dimercapto-3-oxa-5-butylamino-5-oxo-pentanoïque,
- l'acide 2,4-dimercapto-3-oxa-5-[(1-méthyléthyl)amino]-5-oxo-pentanoïque,
- l'acide 2,4-dimercapto-3-oxa-5-[(2-méthylpropyl)amino]-5-oxo-pentanoïque,
- l'acide 2,4-dimercapto-3-oxa-5-[(3-méthylbutyl)amino]-5-oxo-pentanoïque,
- l'acide 2, 4 - dimercapto - 3-oxa-5 -[(2-hydroxyéthyl)amino]- 5 - oxo - pentanoïque,
- l'acide 2,4-dimercapto-3-oxa-5-[(2-hydroxypropyl)amino]-5-oxo-pentanoïque,
- l'acide 2,4-dimercapto-3-oxa-5-[(2-diméthylaminoéthyl)amino]-5-oxo-pentanoïque,
- l'acide 2,4-dimercapto-3-oxa-5-[(3-diméthylaminopropyl)amino]-5-oxo-pentanoïque,
- l'acide 2,4-dimercapto-3-oxa-pentanedioïque, monométhyl ester,
- l'acide 2,4-dimercapto-3-oxa-pentanedioïque, monoéthyl ester,
- l'acide 2,4-dimercapto-3-oxa-pentanedioïque, monopropyl ester,
- l'acide 2,4-dimercapto-3-oxa-pentanedioïque, mono (1-méthyléthyl) ester,
- le 2,4-dimercapto-3-oxa-pentanediamide,
- le 2,4-dimercapto-3-oxa-N,N'-diméthyl-pentanediamide,
- le 2,4-dimercapto-3-oxa-N,N'-diéthyl-pentanediamide,
- le 2,4-dimercapto-3-oxa-N,N'-bis(2-hydroxyéthyl)-pentanediamide,
- le 2,4-dimercapto-3-oxa-N,N'-bis(2-hydroxypropyl)-pentanediamide,
- l'acide 2,4-dimercapto-3-oxa-pentanedioïque, diméthyl ester,
- l'acide 2,4-dimercapto-3-oxa-pentanedioïque, diéthyl ester,
- l'acide 2,4-dimercapto-3-oxa-pentanedioïque, dipropyl ester,
- l'acide 2,4-dimercapto-3-oxa-pentanedioïque, dibutyl ester,
- l'acide 2,6-dimercapto-4-thia-7-méthylamino-7-oxo-heptanedioïque,
- l'acide 2,6-dimercapto-4-thia-7-éthylamino-7-oxo-heptanedioïque,
- l'acide 2,6-dimercapto-4-thia-7-propylamino-7-oxo-heptanedioïque,
- l'acide 2,6-dimercapto-4-thia-7-[(2-hydroxyéthyl)amino]-7-oxo-heptanedioïque,
- l'acide 2,6-dimercapto-4-thia-7-[(2-hydroxypropyl)amino]-7-oxo-heptanedioïque,
- l'acide 2,6-dimercapto-4-thia-7-[(2-diméthylaminoéthyl)amino]-7-oxo-heptanedioïque,
- l'acide 2,6-dimercapto-4-thia-7-[(3-diméthylaminopropyl)amino]-7-oxo-heptanedioïque,
- l'acide 2,6-dimercapto-4-thia-heptanedioïque, monométhyl ester,
- l'acide 2,6-dimercapto-4-thia-heptanedioïque, monoéthyl ester,
- l'acide 2,6-dimercapto-4-thia-heptanedioïque, monopropyl ester,
- le 2,6-dimercapto-4-thia-heptanediamide,
- le 2,6-dimercapto-4-thia-N,N'-diméthyl-heptanediamide,
- le 2,6-dimercapto-4-thia-N,N'-diéthyl-heptanediamide,
- le 2,6-dimercapto-4-thia-N,N'-bis(2-hydroxyéthyl)-heptanediamide,
- le 2,6-dimercapto-4-thia-N,N'-bis(2-hydroxypropyl)-heptanediamide,
- l'acide 2,6-dimercapto-4-thia-heptanedioïque, diméthylester,
- l'acide 2,6-dimercapto-4-thia-heptanedioïque, diéthylester,
- l'acide 2,6-dimercapto-4-thia-heptanedioïque, dipropylester,
- l'acide N-(carboxy-mercapto-méthyl)-2-mercapto-malonamique,
- l'acide [(carboxy-mercapto-méthyl)-méthyl-amino]-mercapto-acétique.

On préfère tout particulièrement les composés suivants :
- l'acide 2,3-dimercapto-4-méthylamino-4-oxo-butanoïque,
- l'acide 2,3-dimercapto-4-éthylamino-4-oxo-butanoïque,
- l'acide 2,3-dimercapto-4-propylamino-4-oxo-butanoïque,
- l'acide 2,3-dimercapto-4-butylamino-4-oxo-butanoïque,
- l'acide 2,3-dimercapto-4-[(2-hydroxyéthyl)amino]-4-oxo-butanoïque,
- l'acide 2,3-dimercapto-4-[(2-hydroxypropyl)amino]-4-oxo-butanoïque,
- l'acide 2,3-dimercapto-4-[(2-diméthylaminoéthyl)amino]-4-oxo-butanoïque,
- l'acide 2,3-dimercapto-4-[(3-diméthylaminopropyl)amino]-4-oxo-butanoïque,
- le 2,3dimercapto-butanediamide,
- le 2,3dimercapto-N,N'-diméthyl-butanediamide,
- le 2,3dimercapto-N,N'-bis(2-hydroxyéthyl)-butanediamide,
- le 2,3dimercapto-N,N'-bis(2-hydroxypropyl)-butanediamide,
- l'acide 2,4-dimercapto-5-méthylamino-5-oxo-pentanoïque,
- l'acide 2,4-dimercapto-5-éthylamino-5-oxo-pentanoïque,
- l'acide 2,4-dimercapto-5-propylamino-5-oxo-pentanoïque,
- l'acide 2,4-dimercapto-5-[(2-hydroxyéthyl)amino]-5-oxo-pentanoïque,
- l'acide 2,4-dimercapto-5-[(2-hydroxypropyl)amino]-5-oxo-pentanoïque,
- l'acide 2,4-dimercapto-5-[(2-diméthylaminoéthyl)amino]-5-oxo-pentanoïque,
- l'acide 2,4-dimercapto-5-[(3-diméthylaminopropyl)amino]-5-oxo-pentanoïque,
- le 2,4-dimercapto-pentanediamide,
- le 2,4-dimercapto-N,N'-diméthyl-pentanediamide,
- le 2,4-dimercapto-N,N'-bis(2-hydroxyéthyl)-pentanediamide,
- le 2,4-dimercapto-N,N'-bis(2-hydroxypropyl)-pentanediamide,
- l' acide 5-carbamoyl-2,5-dimercapto-valérique,
- l'acide 2,5-dimercapto-6-méthylamino-6-oxo-hexanoïque,
- l'acide 2,5-dimercapto-6-éthylamino-6-oxo-hexanoïque,
- l'acide 2,5-dimercapto-6-[(2-hydroxyéthyl)amino]-6-oxo-hexanoïque,
- l'acide 2,5-dimercapto-6-[(2-hydroxypropyl)amino]-6-oxo-hexanoïque,
- l'acide 2,5-dimercapto-6-[(2-diméthylaminoéthyl)amino]-6-oxo-hexanoïque,
- l'acide 2,5-dimercapto-6-[(3-diméthylaminopropyl)amino]-6-oxo-hexanoïque,
- le 2,5-dimercapto-hexanediamide,
- le 2,5-dimercapto-N,N'-diméthyl-hexanediamide,
- le 2,5-dimercapto-N,N'-diéthyl-hexanediamide,
- le 2,5-dimercapto-N,N'-bis(2-hydroxyéthyl)-hexanediamide,
- le 2,5-dimercapto-N,N'-bis(2-hydroxypropyl)-hexanediamide,
- l'acide 2,6-dimercapto-7-méthylamino-7-oxo-heptanedioïque,
- l'acide 2,6-dimercapto-7-éthylamino-7-oxo-heptanedioïque,
- l'acide 2,6-dimercapto-7-[(2-hydroxyéthyl)amino]-7-oxo-heptanedioïque,
- l'acide 2,6-dimercapto-7-[(2-hydroxypropyl)amino]-7-oxo-heptanedioïque,
- le 2,6-dimercapto-heptanediamide,
- le 2,6-dimercapto-N,N'-diméthyl-heptanediamide,
- le 2,6-dimercapto-N,N'-bis(2-hydroxyéthyl)-heptanediamide,
- le 2,6-dimercapto-N,N'-bis(2-hydroxypropyl)-heptanediamide,
- l'acide 2,7-dimercapto-8-méthylamino-8-oxo-octanedioïque,
- l'acide 2,7-dimercapto-8-éthylamino-8-oxo-octanedioïque,
- l'acide 2,7-dimercapto-8-[(2-hydroxyéthyl)amino]-8-oxo-octanedioïque,
- l'acide 2,7-dimercapto-8-[(2-hydroxypropyl)amino]-8-oxo-octanedioïque,
- le 2,8-dimercapto-heptanediamide,
- le 2,8-dimercapto-N,N'-diméthyl-heptanediamide,
- le 2,8-dimercapto-N,N'-bis(2-hydroxyéthyl)-heptanediamide,
- le 2,8-dimercapto-N,N'-bis(2-hydroxypropyl)-heptanediamide,
- l'acide 2,4-dimercapto-3-oxa-5-méthylamino-5-oxo-pentanoïque,
- l'acide 2,4-dimercapto-3-oxa-5-éthylamino-5-oxo-pentanoïque,
- l'acide 2,4-dimercapto-3-oxa-5-[(2-hydroxyéthyl)amino]-5-oxo-pentanoïque,
- l'acide 2,4-dimercapto-3-oxa-5-[(2-hydroxypropyl)amino]-5-oxo-pentanoïque,
- l'acide 2,4-dimercapto-3-oxa-5-[(2-diméthylaminoéthyl)amino]-5-oxo-pentanoïque,
- l'acide 2,4-dimercapto-3-oxa-5-[(3-diméthylaminopropyl)amino]-5-oxo - pentanoïque,
- le 2,4-dimercapto-3-oxa-pentanediamide,
- le 2,4-dimercapto-3-oxa-N,N'-diméthyl-pentanediamide,
- le 2,4-dimercapto-3-oxa-N,N'-bis (2-hydroxyéthyl)-pentanediamide,
- le 2,4-dimercapto-3-oxa-N,N'-bis (2-hydroxypropyl)-pentanediamide,
- l'acide 2,6-dimercapto-4-thia-7-méthylamino-7-oxo-heptanedioïque,
- l'acide 2,6-dimercapto-4-thia-7-éthylamino-7-oxo-heptanedioïque,
- l'acide 2,6-dimercapto-4-thia-7-[(2-hydroxyéthyl)amino]-7-oxo-heptanedioïque,
- l'acide 2,6-dimercapto-4-thia-7-[(2-hydroxypropyl)amino]-7-oxo-heptanedioïque,
- le 2,6-dimercapto-4-thia-heptanediamide,
- le 2,6-dimercapto-4-thia-N,N'-diméthyl-heptanediamide,
- le 2,6-dimercapto-4-thia-N,N'-diéthyl-heptanediamide,
- le 2,6-dimercapto-4-thia-N,N'-bis(2-hydroxyéthyl)-heptanediamide,
- le 2,6-dimercapto-4-thia-N,N'-bis (2-hydroxypropyl)-heptanediamide.

Dans la composition réductrice selon l'invention, le ou les agents réducteurs de formule générale (I) et/ou (II), et/ou leurs sels organiques et minéraux, sont généralement présents à une teneur comprise entre 0,05 et 30 %, de préférence entre 1 et 20 %, en poids par rapport au poids total de la composition réductrice.

Le pH de la composition selon l'invention est de préférence compris entre 4 et 11, et plus particulièrement entre 6 et 10.

Le pH est généralement obtenu à l'aide d'un agent alcalin tel que, par exemple, l'ammoniaque, la monoéthanolamine, la diéthanolamine, la triéthanolamine, la propanediamine-1,3, un carbonate ou bicarbonate alcalin ou d'ammonium, un carbonate organique tel que le carbonate de guanidine, un hydroxyde alcalin, ou à l'aide d'un agent acidifiant tel que par exemple l'acide chlorhydrique, l'acide acétique, l'acide lactique, l'acide oxalique ou l'acide borique, ou bien encore à l'aide de tampons usuels, tels que par exemple les tampons borate, phosphate ou TRIS (à base de tris(hydroxyméthyl) amino méthane).

La composition réductrice selon l'invention peut également contenir un ou plusieurs autres agents réducteurs connus, tels que par exemple l'acide thioglycolique ou l'acide thiolactique et leurs dérivés esters et amides, notamment le monothioglycolate de glycérol, la cystéamine et ses dérivés (Ci-C₄) acylés tels que la N-acétyl-cystéamine ou la N-propionyl-cystéamine, la cystéine, la N-acétyl-cystéine, l'acide thiomalique, la panthétéine, l'acide 2,3-dimercaptosuccinique, les sulfites ou bisulfites d'un métal alcalin ou alcalino-terreux, les N-(mercaptoatkyt)-_{ω-}hydroxyatkytamides tels que ceux décrits dans la demande de brevet EP-A-354 835, les N-mono ou N,N-dialkylmercapto-4-butyramides tels que ceux décrits dans la demande de brevet EP-A-368 763, les aminomercaptoalkylamides, tels que ceux décrits dans la demande de brevet EP-A-432 000, les dérivés des acides N-(mercaptoalkyl)succinamiques et des N-(mercaptoalkyl)succinimides tels que ceux décrits dans la demande de brevet EP-A-465 342, les alkylamino mercaptoalkylamides tels que ceux décrits dans la demande de brevet EP-A-514 282, le mélange azéotrope de thioglycolate de 2-hydroxypropyle et de thioglycolate de (2-hydroxy-1-méthyl)éthyle décrits dans la demande de brevet FR-A-2 679 448, les mercaptoalkylaminoamides tels que ceux décrits dans la demande de brevet FR-A-2 692 481, les N-mercaptoalkylalcanediamides décrits dans la demande de brevet EP-A-653 202, ainsi que les dérivés d'acide formamidine sulfinique tels que ceux décrits dans la demande PCT/US01/43124, déposée par la Demanderesse.

Selon un mode de réalisation préféré, la composition réductrice selon l'invention contient également au moins un agent tensioactif de type non-ionique, anionique, cationique ou amphotère. Parmi ceux-ci, on peut citer les alkyl sulfates, les alkyl benzènesulfates, les alkyl éthersulfates, les alkyl sulfonates, les sels d'ammonium quaternaire, les alkyl bétaïnes, les alkyl phénols oxyéthylénés, les alcanolamides d'acides gras, les esters d'acides gras oxyéthylénés ainsi que d'autres tensioactifs non-ioniques du type hydroxypropyléthers.

Lorsque la composition réductrice selon l'invention contient un ou plusieurs agents tensioactifs, celui-ci ou ceux-ci représentent généralement au plus 30 % en poids, et de préférence entre 0,5 et 10 % en poids, par rapport au poids total de la composition réductrice.

Dans le but d'améliorer les propriétés cosmétiques des cheveux ou encore d'en atténuer ou d'éviter leur dégradation, la composition réductrice peut également contenir un agent traitant de nature cationique, anionique, non-ionique ou amphotère.

Parmi les agents traitants particulièrement préférés, on peut notamment citer ceux décrits dans les brevets français FR 2 598 613 et FR 2 470 596. On peut également utiliser comme agents traitants des silicones volatiles ou non, linéaires ou cycliques, et leurs mélanges, les polydiméthylsiloxanes, les polyorganosiloxanes quaternisés tels que ceux décrits dans la demande de brevet français FR 2 535 730, les polyorganosiloxanes à groupements aminoalkyles modifiés par des groupements alcoxycarbonylalkyles tels que ceux décrits dans le brevet US 4,749,732, des polyorganosiloxanes tels que le copolymère polydiméthylsiloxane-polyoxyalkyle du type diméthicone copolyol, un polydiméthylsiloxane à groupements terminaux stéaroxy (stéaroxy-diméthicone), un copolymère polydiméthylsiloxane dialkylammonium acétate ou un copolymère polydiméthyl-siloxane polyalkylbétaïne décrits dans le brevet britannique n° 2 197 352, des polysiloxanes organo modifiés par des groupements mercapto ou mercaptoalkyles tels que ceux décrits dans le brevet français FR 1 530 369 et dans la demande de brevet européen EP 295 780, ainsi que des silanes tels que le stéaroxytriméthylsilane.

La composition réductrice selon l'invention peut également contenir un ou plusieurs autres ingrédients traitants tels que des polymères cationiques tels que ceux utilisés dans les compositions des brevets français n° 79.32078 (FR 2 472 382) et 80.26421 (FR 2 495 931), ou encore des polymères cationiques du type ionène tels que ceux utilisés dans les compositions du brevet luxembourgeois n°83703, des aminoacides basiques tels que la lysine ou l'arginine, des aminoacides, acides tels que l'acide glutamique ou l'acide aspartique, des peptides et leurs dérivés, des hydrolysats de protéines, des cires, des agents de gonflement et de pénétration, ou des agents permettant de renforcer l'efficacité du réducteur tels que le mélange Si0₂/PDMS (polydiméthylsiloxane), le diméthylisosorbitol, l'urée et ses dérivés, la pyrrolidone, les N-alkyl-pyrrolidones, la thiamorpholinone, les alkyléthers d'alkylèneglycol ou de dialkylèneglycol tels que par exemple le monométhyléther de propylèneglycol, le monométhyléther de dipropylèneglycol, le monoéthyléther de l'éthylèneglycol et le monoéthyléther du diéthylèneglycol, des alcanediols en C₃-C₆ tels que par exemple le propanediol-1,2, le propanediol-1,3 et le butanediol-1,2, l'imidazolidinone-2 ainsi que d'autres composés tels que des alcools gras, des dérivés de la lanoline, des ingrédients actifs tels que l'acide panthothénique, des agents antichute, des agents antipelliculaires, des épaississants, des agents de suspension, des agents séquestrants, des agents opacifiants, des colorants, des filtres solaires ainsi que des parfums et des conservateurs.

Le ou les dicarboxydithiols de formule (I) et/ou (II), et/ou leurs sels organiques et minéraux, doivent être placés dans un milieu cosmétiquement acceptable.

Ainsi, la composition réductrice selon l'invention peut conteni un solvant tel que par exemple de l'éthanol, du propanol ou de l'isopropanol ou encore du glycérol, à une concentration maximale de 20 % en poids par rapport au poids total de la composition.

Le véhicule des compositions selon l'invention est, de préférence, de l'eau ou une solution hydroalcoolique d'un alcool inférieur en C₁-C₆, tel que l'éthanol, l'isopropanol ou le butanol.

De préférence, la composition réductrice selon l'invention se présente essentiellement sous forme aqueuse, notamment sous la forme d'une lotion épaissie ou non, d'une crème épaisse ou non, ou d'un gel.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables ou d'alcools gras.

On peut également utiliser des liquides ou des gels contenant des agents épaississants tels que des polymères ou des copolymères carboxyvinyliques qui "collent" les cheveux et les maintiennent dans la position lisse pendant le temps de pose.

La composition réductrice selon l'invention peut être également du type exothermique, c'est-à-dire provoquant un certain échauffement lors de l'application sur les cheveux, ce qui apporte un agrément à la personne qui subit le premier temps de la permanente ou du défrisage.

Les dicarboxydithiols de formule générale (1) ou (11) sont pour certains nouveaux et leur procédé de préparation sera décrit plus en détail ci-après. Les composés connus sont généralement préparés suivant les modes opératoires décrits dans les références suivantes :
- *Polyhedron* **1999, 18** (25), 3257-3262,
- *Chemical Research in Toxicology* **1996,** 9(6), 965-969,
- *Forschungszentrum* Rossendorf *e.V.*; **1996,** 102-104,
- *Drug and Chemical Toxicology* **1994**, 17(1),69-73,
- *Tetrahedron letters* **1992,** 33(21), 2961-4,
- *Fundamental and Applied Toxicology* **1995,** 26(2), 239-45,
- *Toxicology* **1992,** 76(1), 79-87,
- *J. Org. Chem.* **1991,** 56(26), 7328-7331,
- *Methods in Enzymology* **1995,** 251 (Biothiols, part A), 167-173,
- US 2930799,
- *J. Org. Chem.* **1981,** 46(10), 2072-9.

L'invention concerne encore un procédé de déformation permanente des cheveux mettant en oeuvre une composition réductrice comprenant au moins un composé de formule (1) et/ou (11), et/ou au moins un sel organique ou minéral dudit composé.

Le procédé de déformation permanente des cheveux selon l'invention comprend :
- une étape d'application sur les cheveux d'une composition réductrice selon l'invention, pour réduire les liaisons disulfures de la kératine ;
- une étape de fixation par oxydation, pour refermer lesdites liaisons, par application d'une composition oxydante sur les cheveux ou par mise en contact des cheveux avec l'oxygène de l'air.

Les cheveux sont mis en forme en utilisant des moyens mécaniques bien connus de l'homme de l'art.

Lorsque l'on souhaite réaliser une permanente, on utilise de préférence des bigoudis, la composition réductrice étant appliquée avant ou après les moyens de mise en forme des cheveux, et la composition oxydante de fixation étant appliquée après la composition réductrice, avec ou sans étape intermédiaire ou subséquente de rinçage ou d'application de composition intermédiaire.

De préférence, on applique une composition réductrice selon l'invention sur des cheveux mouillés préalablement enroulés sur des rouleaux ayant de 2 à 30 mm de diamètre. La composition peut également être appliquée au fur et à mesure de l'enroulage des cheveux. Généralement, on laisse ensuite agir la composition réductrice pendant un temps de 5 à 60 minutes, de préférence de 15 à 45 minutes, puis on rince abondamment. On applique alors, sur les cheveux enroulés, la composition oxydante permettant de reformer les liaisons disulfures de la kératine, pendant un temps de pose de 2 à 10 minutes. Après avoir enlevé les rouleaux, on rince abondamment la chevelure.

On peut également, après application de la composition réductrice, soumettre la chevelure à un traitement thermique par chauffage à une température comprise entre 30 et 60°C pendant tout ou partie du temps de pose. Dans la pratique, cette opération peut être conduite au moyen d'un casque de coiffure, d'un sèche cheveux, d'un dispensateur de rayons infrarouges et d'autres appareils chauffants classiques.

On peut notamment utiliser, à la fois comme moyen de chauffage et de mise en forme de la chevelure, un fer chauffant à une température comprise entre 60 et 220°C et de préférence entre 120 et 200°C, l'utilisation du fer chauffant se faisant entre l'étape d'application de la composition réductrice et l'étape de fixation.

Lorsque l'on souhaite effectuer le défrisage ou le décrêpage des cheveux, on applique sur les cheveux une composition réductrice selon l'invention, puis l'on soumet les cheveux à une déformation mécanique permettant de les fixer dans leur nouvelle forme, par une opération de lissage des cheveux avec un peigne à larges dents, avec le dos d'un peigne ou à la main. Après généralement un temps de pose de 5 à 60 minutes, de préférence de 15 à 45 minutes, on procède à un nouveau lissage, puis on rince soigneusement et on applique la composition oxydante telle que définie ci-dessus, qu'on laisse agir pendant environ 2 à 10 minutes, puis on rince abondamment les cheveux.

La composition oxydante peut être toute composition oxydante couramment utilisée pour la déformation permanente des cheveux et contient un agent oxydant choisi de préférence parmi l'eau oxygénée, les bromates alcalins, les persels, les polythionates et un mélange de bromate alcalin et de persel. La concentration en eau oxygénée peut varier de 1 à 20 volumes et de préférence de 1 à 10, la concentration en bromate alcalin de 2 à 12 % et celle en persel de 0,1 à 15 %, en poids par rapport au poids total de la composition oxydante.

Le pH de la composition oxydante est généralement compris entre 2 et 10.

Comme expliqué précédemment, l'application de la composition oxydante peut être effectuée immédiatement ou être différée.

Selon un mode de réalisation particulier du procédé selon l'invention, le lissage des cheveux peut également être effectué, en tout ou partie, à l'aide d'un fer chauffant entre 60 et 220°C, et de préférence entre 120 et 200°C.

L'invention concerne également un kit, notamment pour la déformation permanente des cheveux comprenant, dans un premier compartiment, en tant que composition réductrice, une composition réductrice selon l'invention comprenant un ou plusieurs composés de formule (1) et/ou (11) tels que définis précédemment et/ou un ou plusieurs de leurs sels organiques et minéraux, et dans un second compartiment, une composition oxydante.

Généralement, la composition oxydante comprend au moins un agent oxydant choisi parmi l'eau oxygénée, les bromates alcalins, les persels, les polythionates et un mélange de bromate alcalin et de persel.

La présente invention a également pour objet, à titre de composé nouveau, un dicarboxydithiol répondant à la formule générale (III) ou à la formule générale (IV) suivantes : 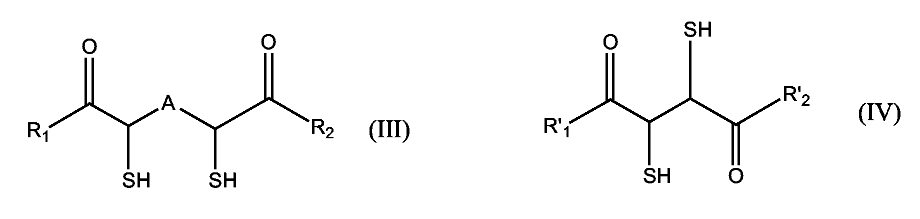 dans lesquelles :
A, R₁, R₂, R'₁ et R'₂ ont les mêmes significations que précédemment, à l'exclusion des composés dans lesquels :
   (i) R'₁ représente OH et R'₂ représente OR₃, R₃ étant un radical alkyle linéaire ou ramifié en C₁-C₅, ou bien R'₂ représente NR₄R₅, R₄ étant un atome d'hydrogène et R₅ un radical alkyle ramifié en C₃-C₅ ;
   (ii) R'₁ et R'₂ représentent un radical amino ;
   (iii) R'₁ et R'₂ représentent le radical OR₃ dans lequel R₃ désigne un radical alkyle linéaire ou ramifié en C₁-C₅ ainsi que les radicaux 2-méthoxyéthyle et 2-éthoxyéthyle ;
   (iv) A représente CH₂ et R₁ et R₂ représentent un radical amino ;
   (v) A représente CH₂ et R₁ et R₂ représentent le radical OR₃ dans lequel R₃ désigne un radical butyle ;
   (vi) A représente (CH₂)₂, R₁ représente OH et R₂ représente NR₄R₅, R₄ étant un atome d'hydrogène et R₅ un atome d'hydrogène ;
   (vii) A représente (CH₂)₂ et R₁ et R₂ représentent un radical diméthylamino ;
   (viii) A représente (CH₂)₂ et R₁ et R₂ représentent le radical OR₃ dans lequel R₃ désigne un radical méthyle ;
ou un sel organique ou minéral dudit composé de formule (III) ou (IV).

Parmi les composés de formule générale (III) ou (IV), on peut notamment citer les composés préférés ci-après :
- l'acide 2,3-dimercapto-4-méthylamino-4-oxo-butanoïque,
- l'acide 2,3-dimercapto-4-éthylamino-4-oxo-butanoïque,
- l'acide 2,3-dimercapto-4-propylamino-4-oxo-butanoïque,
- l'acide 2,3-dimercapto-4-butylamino-4-oxo-butanoïque,
- l'acide 2,3-dimercapto-4-[(2-hydroxyéthyl)amino]-4-oxo-butanoïque,
- l'acide 2,3-dimercapto-4-[(2-hydroxypropyl)amino]-4-oxo-butanoïque,
- l'acide 2,3-dimercapto-4-[(2-diméthylaminoéthyl)amino]-4-oxo-butanoïque,
- l'acide 2,3-dimercapto-4-[(3-diméthylaminopropyl)amino]-4-oxo-butanoïque,
- le 2,3dimercapto-N,N'-diméthyl-butanediamide,
- le 2,3dimercapto-N,N'-diéthyl-butanediamide,
- le 2,3dimercapto-N,N'-bis(2-hydroxyéthyl)-butanediamide,
- le 2,3dimercapto-N,N'-bis(2-hydroxypropyl)-butanediamide,
- l'acide 2,4-dimercapto-5-méthylamino-5-oxo-pentanoïque,
- l'acide 2,4-dimercapto-5-éthylamino-5-oxo-pentanoïque,
- l'acide 2,4-dimercapto-5-propylamino-5-oxo-pentanoïque,
- l'acide 2,4-dimercapto-5-butylamino-5-oxo-pentanoïque,
- l'acide 2,4-dimercapto-5-[(1-méthyléthyl)amino]-5-oxo-pentanoïque,
- l'acide 2,4-dimercapto-5-[(2-méthylpropyl)amino]-5-oxo-pentanoïque,
- l'acide 2,4-dimercapto-5-[(3-méthylbutyl)amino]-5-oxo-pentanoïque,
- l'acide 2,4-dimercapto-5-[(2-hydroxyéthyl)amino]-5-oxo-pentanoïque,
- l'acide 2,4-dimercapto-5-[(2-hydroxypropyl)amino]-5-oxo-pentanoïque,
- l'acide 2,4-dimercapto-5-[(2-diméthylaminoéthyl)amino]-5-oxo-pentanoïque,
- l'acide 2,4-dimercapto-5-[(3-diméthylaminopropyl)amino]-5-oxo-pentanoïque,
- l'acide 2,4-dimercapto-pentanedioïque, monométhyl ester,
- l'acide 2,4-dimercapto-pentanedioïque, monoéthyl ester,
- l'acide 2,4-dimercapto-pentanedioïque, monopropyl ester,
- l'acide 2,4-dimercapto-pentanedioïque, mono(1-méthyléthyl)ester,
- le 2,4-dimercapto-N,N'-diméthyl-pentanediamide,
- le 2,4-dimercapto-N,N'-diéthyl-pentanediamide,
- le 2,4-dimercapto-N,N'-bis(2-hydroxyéthyl)-pentanediamide,
- le 2,4-dimercapto-N,N'-bis (2-hydroxypropyl)-pentanediamide,
- l'acide 2,4-dimercapto-pentanedioïque, diméthyl ester,
- l'acide 2,4-dimercapto-pentanedioïque, diéthyl ester,
- l'acide 2,4-dimercapto-pentanedioïque, dipropyl ester,
- l'acide 2,5-dimercapto-6-méthylamino-6-oxo-hexanoïque,
- l'acide 2,5-dimercapto-6-éthylamino-6-oxo-hexanoïque,
- l'acide 2,5-dimercapto-6-propylamino-6-oxo-hexanoïque,
- l'acide 2,5-dimercapto-6-[(2-hydroxyéthyl)amino]-6-oxo-hexanoïque,
- l'acide 2,5-dimercapto-6-[(2-hydroxypropyl)amino]-6-oxo-hexanoïque,
- l'acide 2,5-dimercapto-6-[(2-diméthylaminoéthyl)amino]-6-oxo-hexanoïque,
- l'acide 2,5-dimercapto-6-[(3-diméthylaminopropyl)amino]-6-oxo-hexanoïque
- l'acide 2,5-dimercapto-hexanedioïque, monométhyl ester,
- l'acide 2,5-dimercapto-hexanedioïque, monoéthyl ester,
- l'acide 2,5-dimercapto-hexanedioïque, monopropyl ester,
- le 2,5-dimercapto-hexanediamide,
- le 2,5-dimercapto-N,N'-diméthyl-hexanediamide,
- le 2,5-dimercapto-N,N'-diéthyl-hexanediamide,
- le 2,5-dimercapto-N,N'-bis(2-hydroxyéthyl)-hexanediamide,
- le 2,5-dimercapto-N,N'-bis(2-hydroxypropyl)-hexanediamide,
- l'acide 2,5-dimercapto-hexanedioïque, diéthylester,
- l'acide 2,5-dimercapto-hexanedioïque, dipropylester,
- l'acide 2,6-dimercapto-7-méthylamino-7-oxo-heptanedioïque,
- l'acide 2,6-dimercapto-7-éthylamino-7-oxo-heptanedioïque,
- l'acide 2,6-dimercapto-7-propylamino-7-oxo-heptanedioïque,
- l'acide 2,6-dimercapto-7-[(2-hydroxyéthyl)amino]-7-oxo-heptanedioïque,
- l'acide 2,6-dimercapto-7-[(2-hydroxypropyl)amino]-7-oxo-heptanedioïque,
- l'acide 2,6-dimercapto-7-[(2-diméthylaminoéthyl)amino]-7-oxo-heptanedioïque,
- l'acide 2,6-dimercapto-7-[(3-diméthylaminopropyl)amino]-7-oxo-heptanedioïque,
- l'acide 2,6-dimercapto-heptanedioïque, monométhyl ester,
- l'acide 2,6-dimercapto-heptanedioïque, monoéthyl ester,
- l'acide 2,6-dimercapto-heptanedioïque, monopropyl ester,
- le 2,6-dimercapto-heptanediamide,
- le 2,6-dimercapto-N,N'-diméthyl-heptanediamide,
- le 2,6-dimercapto-N,N'-bis (2-hydroxyéthyl)-heptanediamide,
- le 2,6-dimercapto-N,N'-bis (2-hydroxypropyl)-heptanediamide,
- l'acide 2,6-dimercapto-heptanedioïque, diméthylester,
- l'acide 2,6-dimercapto-heptanedioïque, diéthylester,
- l'acide 2,6-dimercapto-heptanedioïque, dipropylester,
- l'acide 2,7-dimercapto-8-méthylamino-8-oxo-octanedioïque,
- l'acide 2,7-dimercapto-8-éthylamino-8-oxo-octanedioïque,
- l'acide 2,7-dimercapto-8-propylamino-8-oxo-octanedioïque,
- l'acide 2,7-dimercapto-8-[(2-hydroxyéthyl)amino]-8-oxo-octanedioïque,
- l'acide 2,7-dimercapto-8-[(2-hydroxypropyl)amino]-8-oxo-octanedioïque,
- l'acide 2,7-dimercapto-8-[(2-diméthylaminoéthyl)amino]-8-oxo-octanedioïque,
- l'acide 2,7-dimercapto-8-[(3-diméthylaminopropyl)amino]-8-oxo-octanedioïque,
- l'acide 2,8-dimercapto-octanedioïque, monométhyl ester,
- l'acide 2,8-dimercapto-octanedioïque, monoéthyl ester,
- l'acide 2,8-dimercapto-octanedioïque, monopropyl ester,
- le 2,8-dimercapto-heptanediamide,
- le 2,8-dimercapto-N,N'-diméthyl-heptanediamide,
- le 2,8-dimercapto-N,N'-bis(2-hydroxyéthyl)-heptanediamide,
- le 2,8-dimercapto-N,N'-bis(2-hydroxypropyl)-heptanediamide,
- l'acide 2,8-dimercapto-heptanedioïque, diméthylester,
- l'acide 2,8-dimercapto-heptanedioïque, diéthylester,
- l'acide 2,8-dimercapto-heptanedioïque, dipropylester,
- l'acide 2,4-dimercapto-3-oxa-5-méthylamino-5-oxo-pentanoïque,
- l'acide 2,4-dimercapto-3-oxa-5-éthylamino-5-oxo-pentanoïque,
- l'acide 2,4-dimercapto-3-oxa-5-propylamino-5-oxo-pentanoïque,
- l'acide 2,4-dimercapto-3-oxa-5-butylamino-5-oxo-pentanoïque,
- l'acide 2,4-dimercapto-3-oxa-5-[(1-méthyléthyl)amino]-5-oxo-pentanoïque,
- l'acide 2,4-dimercapto-3-oxa-5-[(2-méthylpropyl)amino]-5-oxo-pentanoïque,
- l'acide 2,4-dimercapto-3-oxa-5-[(3-méthylbutyl)amino]-5-oxo-pentanoïque,
- l'acide 2,4-dimercapto-3-oxa-5-[(2-hydroxyéthyl)amino]-5-oxo-pentanoïque,
- l'acide 2,4-dimercapto-3-oxa-5-[(2-hydroxypropyl)amino]-5-oxo-pentanoïque,
- l'acide 2,4-dimercapto-3-oxa-5-[(2-diméthylaminoéthyl)amino]-5-oxo-pentanoïque,
- l'acide 2,4-dimercapto-3-oxa-5-[(3-diméthylaminopropyl)amino]-5-oxo - pentanoïque,
- l'acide 2,4-dimercapto-3-oxa-pentanedioïque, monométhyl ester,
- l'acide 2,4-dimercapto-3-oxa-pentanedioïque, monoéthyl ester,
- l'acide 2,4-dimercapto-3-oxa-pentanedioïque, monopropyl ester,
- l'acide 2,4-dimercapto-3-oxa-pentanedioïque, mono(1-méthyléthyl) ester,
- le 2,4-dimercapto-3-oxa-pentanediamide,
- le 2,4-dimercapto-3-oxa-N,N'-diméthyl-pentanediamide,
- le 2,4-dimercapto-3-oxa-N,N'-diéthyl-pentanediamide,
- le 2,4-dimercapto-3-oxa-N,N'-bis (2-hydroxyéthyl)-pentanediamide,
- le 2,4-dimercapto-3-oxa-N,N'-bis(2-hydroxypropyl)-pentanediamide,
- l'acide 2,4-dimercapto-3-oxa-pentanedioïque, diméthyl ester,
- l'acide 2,4-dimercapto-3-oxa-pentanedioïque, diéthyl ester,
- l'acide 2,4-dimercapto-3-oxa-pentanedioïque, dipropyl ester,
- l'acide 2,4-dimercapto-3-oxa-pentanedioïque, dibutyl ester,
- l'acide 2,6-dimercapto-4-thia-7-méthylamino-7-oxo-heptanedioïque,
- l'acide 2,6-dimercapto-4-thia-7-éthylamino-7-oxo-heptanedioïque,
- l'acide 2,6-dimercapto-4-thia-7-propylamino-7-oxo-heptanedioïque,
- l'acide 2,6-dimercapto-4-thia-7-[(2-hydroxyéthyl)amino]-7-oxo-heptanedioïque,
- l'acide 2,6-dimercapto-4-thia-7-[(2-hydroxypropyl)amino]-7-oxo-heptanedioïque,
- l'acide 2,6-dimercapto-4-thia-7-[(2-diméthylaminoéthyl)amino]-7-oxo-heptanedioïque,
- l'acide 2,6-dimercapto-4-thia-7-[(3-diméthylaminopropyl)amino]-7-oxo-heptanedioïque,
- l'acide 2,6-dimercapto-4-thia-heptanedioïque, monométhyl ester,
- l'acide 2,6-dimercapto-4-thia-heptanedioïque, monoéthyl ester,
- l'acide 2,6-dimercapto-4-thia-heptanedioïque, monopropyl ester,
- le 2,6-dimercapto-4-thia-heptanediamide,
- le 2,6-dimercapto-4-thia-N,N'-diméthyl-heptanediamide,
- le 2,6-dimercapto-4-thia-N,N'-diéthyl-heptanediamide,
- le 2,6-dimercapto-4-thia-N,N'-bis(2-hydroxyéthyl)-heptanediamide,
- le 2,6-dimercapto-4-thia-N,N'-bis(2-hydroxypropyl)-heptanediamide,
- l'acide 2,6-dimercapto-4-thia-heptanedioïque, diméthylester,
- l'acide 2,6-dimercapto-4-thia-heptanedioïque, diéthylester,
- l'acide 2,6-dimercapto-4-thia-heptanedioïque, dipropylester,
- l'acide N-(carboxy-mercapto-méthyl)-2-mercapto-malonamique,
- l'acide [(carboxy-mercapto-méthyl)-méthyl-amino]-mercapto-acétique.

On préfère tout particulièrement les composés suivants :
- l'acide 2,3-dimercapto-4-méthylamino-4-oxo-butanoïque,
- l'acide 2,3-dimercapto-4-éthylamino-4-oxo-butanoïque,
- l'acide 2,3-dimercapto-4-propylamino-4-oxo-butanoïque,
- l'acide 2,3-dimercapto-4-butylamino-4-oxo-butanoïque,
- l'acide 2,3-dimercapto-4-[(2-hydroxyéthyl)amino]-4-oxo-butanoïque,
- l'acide 2,3-dimercapto-4-[(2-hydroxypropyl)amino]-4-oxo-butanoïque,
- l'acide 2,3-dimercapto-4-[(2-diméthylaminoéthyl)amino]-4-oxo-butanoïque,
- l'acide 2,3-dimercapto-4-[(3-diméthylaminopropyl)amino]-4-oxo-butanoïque,
- le 2,3dimercapto-N,N'-diméthyl-butanediamide,
- le 2,3dimercapto-N,N'-bis (2-hydroxyéthyl)-butanediamide,
- le 2,3dimercapto-N,N'-bis(2-hydroxypropyl)-butanediamide,
- l'acide 2,4-dimercapto-5-méthylamino-5-oxo-pentanoïque,
- l'acide 2,4-dimercapto-5-éthylamino-5-oxo-pentanoïque,
- l'acide 2,4-dimercapto-5-propylamino-5-oxo-pentanoïque,
- l'acide 2,4-dimercapto-5-[(2-hydroxyéthyl)amino]-5-oxo-pentanoïque,
- l'acide 2,4-dimercapto-5-[(2-hydroxypropyl)amino]-5-oxo-pentanoïque,
- l'acide 2,4-dimercapto-5-[(2-diméthylaminoéthyl)amino]-5-oxo-pentanoïque,
- l'acide 2,4-dimercapto-5-[(3-diméthylaminopropyl)amino]-5-oxo-pentanoïque,
- le 2,4-dimercapto-N,N'-diméthyl-pentanediamide,
- le 2,4-dimercapto-N,N'-bis(2-hydroxyéthyl)-pentanediamide,
- le 2,4-dimercapto-N,N'-bis(2-hydroxypropyl)-pentanediamide,
- l'acide 2,5-dimercapto-6-méthylamino-6-oxo-hexanoïque,
- l'acide 2,5-dimercapto-6-éthylamino-6-oxo-hexanoïque,
- l'acide 2,5-dimercapto-6-[(2-hydroxyéthyl)amino]-6-oxo-hexanoïque,
- l'acide 2,5-dimercapto-6-[(2-hydroxypropyl)amino]-6-oxo-hexanoïque,
- l'acide 2,5-dimercapto-6-[(2-diméthylaminoéthyl)amino]-6-oxo-hexanoïque,
- l'acide 2,5-dimercapto-6-[(3-diméthylaminopropyl)amino]-6-oxo-hexanoïque,
- le 2,5-dimercapto-hexanediamide,
- le 2,5-dimercapto-N,N'-diméthyl-hexanediamide,
- le 2,5-dimercapto-N,N'-diéthyl-hexanediamide,
- le 2,5-dimercapto-N,N'-bis(2-hydroxyéthyl)-hexanediamide,
- le 2,5-dimercapto-N,N'-bis(2-hydroxypropyl)-hexanediamide,
- l'acide 2,6-dimercapto-7-méthylamino-7-oxo-heptanedioïque,
- l'acide 2,6-dimercapto-7-éthylamino-7-oxo-heptanedioïque,
- l'acide 2,6-dimercapto-7-[(2-hydroxyéthyl)amino]-7-oxo-heptanedioïque,
- l'acide 2,6-dimercapto-7-[(2-hydroxypropyl)amino]-7-oxo-heptanedioïque,
- le 2,6-dimercapto-heptanediamide,
- le 2,6-dimercapto-N,N'-diméthyl-heptanediamide,
- le 2,6-dimercapto-N,N'-bis(2-hydroxyéthyl)-heptanediamide,
- le 2,6-dimercapto-N,N'-bis(2-hydroxypropyl)-heptanediamide,
- l'acide 2,7-dimercapto-8-méthylamino-8-oxo-octanedioïque,
- l'acide 2,7-dimercapto-8-éthylamino-8-oxo-octanedioïque,
- l'acide 2,7-dimercapto-8-[(2-hydroxyéthyl)amino]-8-oxo-octanedioïque,
- l'acide 2,7-dimercapto-8-[(2-hydroxypropyl)amino]-8-oxo-octanedioïque,
- le 2,8-dimercapto-heptanediamide,
- le 2,8-dimercapto-N,N'-diméthyl-heptanediamide,
- le 2,8-dimercapto-N,N'-bis(2-hydroxyéthyl)-heptanediamide,
- le 2,8-dimercapto-N,N'-bis(2-hydroxypropyl)-heptanediamide,
- l'acide 2,4-dimercapto-3-oxa-5-méthylamino-5-oxo-pentanoïque,
- l'acide 2,4-dimercapto-3-oxa-5-éthylamino-5-oxo-pentanoïque,
- l'acide 2,4-dimercapto-3-oxa-5-[(2-hydroxyéthyl)amino]-5-oxo-pentanoïque,
- l'acide 2,4-dimercapto-3-oxa-5-[(2-hydroxypropyl)amino]-5-oxo-pentanoïque,
- l'acide 2,4-dimercapto-3-oxa-5-[(2-diméthylaminoéthyl)amino]-5-oxo-pentanoïque,
- l'acide 2,4-dimercapto-3-oxa-5-[(3-diméthylaminopropyl)amino]-5-oxo - pentanoïque,
- le 2,4-dimercapto-3-oxa-pentanediamide,
- le 2,4-dimercapto-3-oxa-N,N'-diméthyl-pentanediamide,
- le 2,4-dimercapto-3-oxa-N,N'-bis(2-hydroxyéthyl)-pentanediamide,
- le 2,4-dimercapto-3-oxa-N,N'-bis(2-hydroxypropyl)-pentanediamide,
- l'acide 2,6-dimercapto-4-thia-7-méthylamino-7-oxo-heptanedioïque,
- l'acide 2,6-dimercapto-4-thia-7-éthylamino-7-oxo-heptanedioïque,
- l'acide 2,6-dimercapto-4-thia-7-[(2-hydroxyéthyl)amino]-7-oxo-heptanedioïque,
- l'acide 2,6-dimercapto-4-thia-7-[(2-hydroxypropyl)amino]-7-oxo-heptanedioïque,
- le 2,6-dimercapto-4-thia-heptanediamide,
- le 2,6-dimercapto-4-thia-N,N'-diméthyl-heptanediamide,
- le 2,6-dimercapto-4-thia-N,N'-diéthyl-heptanediamide,
- le 2,6-dimercapto-4-thia-N,N'-bis(2-hydroxyéthyl)-heptanediamide,
- le 2,6-dimercapto-4-thia-N,N'-bis(2-hydroxypropyl)-heptanediamide.

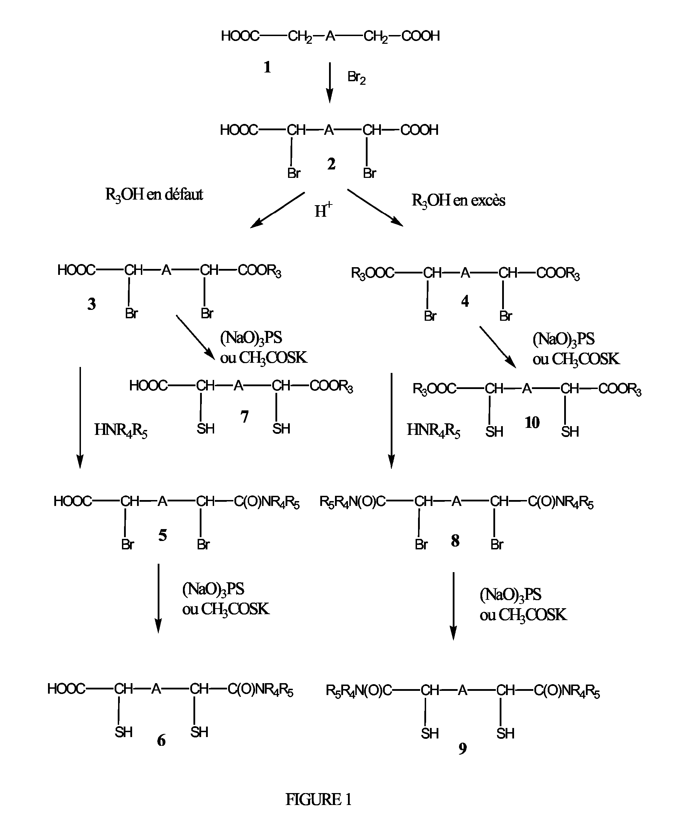

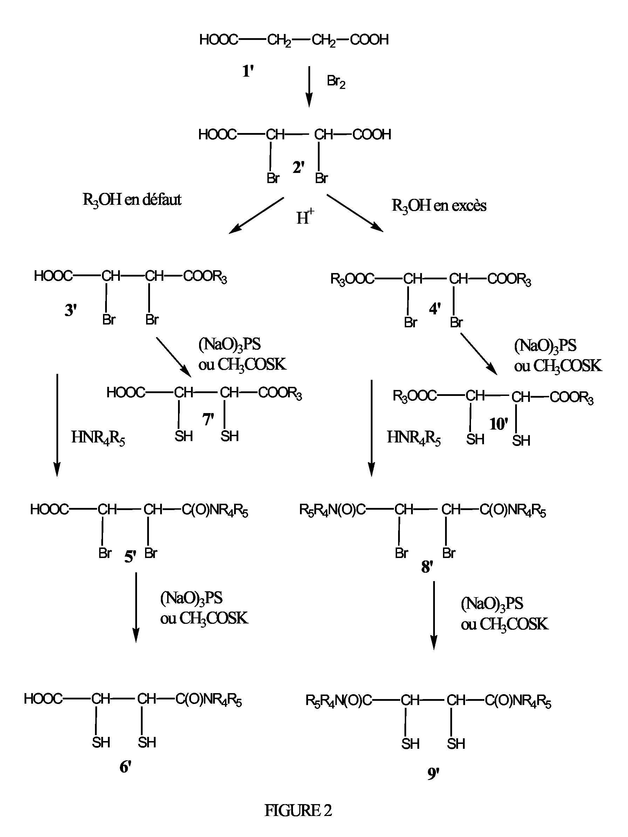

Les composés nouveaux de formule générale (III) et (IV) peuvent être préparés par analogie avec les modes opératoires décrits dans les références citées ci-dessus. Ils peuvent également être préparés selon le schéma de synthèse décrit ci-après, faisant référence aux figures 1 et 2.
La figure 1 représente le schéma de synthèse des composés nouveaux de formule générale (III).
La figure 2 représente le schéma de synthèse des composés nouveaux de formule générale (IV).

On brome le diacide carboxylique de formule (1) (respectivement de formule (1')) dans les conditions habituelles , par exemple en présence de quantités catalytiques de pentachlorure de phosphore ou de chlorure de thionyle pour obtenir l'acide dibromé de formule (2) (respectivement de formule (2')).

Celui-ci est estérifié avec un alcool primaire de formule R₃-OH de façon classique, par exemple en présence de quantités catalytiques d'un acide fort tel que l'acide sulfurique de façon à obtenir les composés de formule (3) et (4) (respectivement de formule (3') et (4')).

On fait réagir un thiophosphate, de préférence le thiophosphate de sodium avec ces composés de formule (3) et (4) (respectivement de formule (3') et (4')) dans un solvant aprotique dipolaire tel que le tétrahydrofurane ou le diméthylformamide, ou encore dans un solvant protique tel que le méthanol, à une température comprise entre la température ambiante et la température de reflux du solvant choisi, puis on hydrolyse avec une solution diluée d'un acide fort tel que l'acide chlorhydrique ou l'acide sulfurique. On peut également faire réagir ces composés de formule (3) et (4) (respectivement de formule (3') et (4')) avec l'acide thioacétique, de préférence son sel de potassium, puis on hydrolyse de préférence en milieu basique. On obtient ainsi respectivement les composés selon l'invention de formule (7) et (10) (respectivement de formule (7') et (10')).

On peut également, à partir des composés de formule (3) et (4) (respectivement de formule (3') et (4')), préparer les amides correspondants de formule (5) et (8) (respectivement de formule (5') et (8')) par réaction avec une amine de formule HNR₄R₅ dans les conditions habituelles de la transformation des esters en amides.

On fait ensuite réagir un thiophosphate, de préférence le thiophosphate de sodium, avec ces composés de formule (5) et (8) dans un solvant aprotique dipolaire tel que le tétrahydrofurane ou le diméthylformamide, ou encore dans un solvant protique tel que le méthanol à une température comprise entre la température ambiante et la température de reflux du solvant choisi puis on hydrolyse avec une solution diluée d'un acide fort tel que l'acide chlorhydrique ou l'acide sulfurique. On peut également faire réagir ces composés de formule (5) et (8) (respectivement de formule (5') et (8')) avec l'acide thioacétique, de préférence son sel de potassium, puis on hydrolyse de préférence en mileu basique.

On obtient ainsi respectivement les composés selon l'invention de formule (6) et (9) (respectivement de formule (6') et (9')).

L'invention pourra être mieux comprise à l'aide des exemples non limitatifs qui suivent et qui constituent des modes de réalisation préférentiels des compositions selon l'invention.

### EXEMPLES DE COMPOSITIONS

### Exemple 1 :

On prépare une composition réductrice de déformation permanente des cheveux selon l'invention en procédant au mélange des composés suivants :
- acide 2,3-dimercapto-4-éthylamino-4-oxo-butanoïque : 12,5g
- ammoniaque à 20% : qsp pH 8,5
- eau déminéralisée : qsp 100 g

Cette composition est appliquée sur des cheveux mouillés préalablement enroulés sur des rouleaux de mise en plis. On laisse agir la composition pendant environ 15 minutes, puis on sèche l'ensemble au sèche-cheveux pendant 5 minutes et on rince abondamment à l'eau.

On applique ensuite la composition oxydante suivante :
- eau oxygénée à 200 volumes : 4,8 g
- acide citrique : qsp pH 3
- eau déminéralisée : qsp 100 g

On laisse agir la composition oxydante pendant 5 minutes puis on rince abondamment à l'eau. On enlève alors les bigoudis et on sèche sous casque. On obtient une belle frisure soutenue et nerveuse.

### Exemple 2 :

On prépare une composition réductrice de déformation permanente des cheveux selon l'invention en procédant au mélange des ingrédients suivants :
- acide 2,3-dimercapto-4-[(2-hydroxypropyl)amino]-4-oxo-butanoïque : 16,7g
- ammoniaque à 20% : qsp pH 8,5
- eau déminéralisée : qsp 100 g

Cette composition est appliquée sur des cheveux mouillés préalablement enroulés sur des rouleaux de mise en plis. On laisse agir la composition pendant environ 15 minutes, puis on sèche l'ensemble au sèche-cheveux pendant 5 minutes et on rince abondamment à l'eau.

On applique ensuite la composition oxydante suivante :
- eau oxygénée à 200 volumes : 4,8 g
- acide citrique : qsp pH 3
- eau déminéralisée : qsp 100 g

On laisse la composition oxydante pendant 5 minutes puis on rince abondamment à l'eau, on enlève les bigoudis et on sèche sous casque. On obtient une belle frisure soutenue et nerveuse.

## Revendications

1. Composition aqueuse réductrice, pour le premier temps d'une opération de déformation permanente des cheveux, contenant, dans un milieu cosmétiquement acceptable, en tant qu'agent réducteur, un ou plusieurs dicarboxydithiols de formule générale (1) et/ou de formule générale (II) : 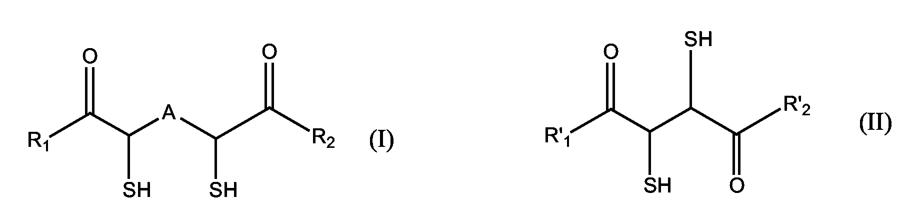 dans lesquelles :
R₁, R₂, R'₁, R'₂ représentent indépendamment OR₃ ou NR₄R₅, où R₃ est H ou un groupe alkyle linéaire ou ramifié en C₁-C₅ éventuellement substitué par un ou deux radicaux OR₇, où R₇ est H ou un radical alcoxy en C₁-C₃,
R₄, R₅ sont indépendamment H ou un groupe alkyle linéaire ou ramifié en C₁-C₅ éventuellement substitué par 1 ou 2 radicaux choisis parmi les radicaux hydroxy, méthoxy, éthoxy, méthylamino, éthylamino et diméthylamino,
R₁ et R₂ ne représentant pas simultanément un radical hydroxyle et R'₁ et R'₂ ne représentant pas simultanément un radical hydroxyle ;
A représente soit (CH₂)ₙ où n est un entier variant de 1 à 4, soit un radical - (CH₂)ₘ-X-(CH₂)ₚ- dans lequel m est égal à 1 ou 2, p est égal à 1 ou 2 et X représente O, S, NHCO ou NR₆, où R₆ représente H ou CH₃,
et/ou un ou plusieurs de leurs sels organiques et minéraux.

2. Composition selon la revendication 1 **caractérisée en ce que** le ou les dicarboxydithiols de formule générale (1) et/ou de formule générale (11) sont choisis parmi :
- l'acide 2,3-dimercapto-4-méthylamino-4-oxo-butanoïque,
- l'acide 2,3-dimercapto-4-éthylamino-4-oxo-butanoïque,
- l'acide 2,3-dimercapto-4-propylamino-4-oxo-butanoïque,
- l'acide 2,3-dimercapto-4-butylamino-4-oxo-butanoïque,
- l'acide 2,3-dimercapto-4-[(1-méthyléthyl)amino]-4-oxo-butanoïque,
- l'acide 2,3-dimercapto-4-[(2-méthylpropyl)amino]-4-oxo-butanoïque,
- l'acide 2,3-dimercapto-4-[(3-méthylbutyl)amino]-4-oxo-butanoïque,
- l'acide 2,3-dimercapto-4-[(2-hydroxyéthyl)amino]-4-oxo-butanoïque,
- l'acide 2,3-dimercapto-4-[(2-hydroxypropyl)amino]-4-oxo-butanoïque,
- l'acide 2,3-dimercapto-4-[(2-diméthylaminoéthyl)amino]-4-oxo-butanoïque,
- l'acide 2,3-dimercapto-4-[(3-diméthylaminopropyl)amino]-4-oxo-butanoïque,
- l'acide 2,3-dimercapto-butanedioïque, monométhyl ester,
- l'acide 2,3-dimercapto-butanedioïque, monoéthyl ester,
- l'acide 2,3-dimercapto-butanedioïque, monopropyl ester,
- l'acide 2,3-dimercapto-butanedioïque, mono(1-méthyléthyl)ester,
- l'acide 2,3-dimercapto-butanedioïque, monobutyl ester,
- l'acide 2,3-dimercapto-butanedioïque, mono(2-méthylpropyl)ester,
- l'acide 2,3-dimercapto-butanedioïque, monopentyl ester,
- l'acide 2,3-dimercapto-butanedioïque, mono(3-méthylbutyl)ester,
- le 2,3dimercapto-butanediamide,
- le 2,3dimercapto-N,N'-diméthyl-butanediamide,
- le 2,3dimercapto-N,N'-diéthyl-butanediamide,
- le 2,3dimercapto-N,N'-bis(2-hydroxyéthyl)-butanediamide,
- le 2,3dimercapto-N,N'-bis(2-hydroxypropyl)-butanediamide,
- l'acide 2,3-dimercapto-butanedioïque, diméthyl ester,
- l'acide 2,3-dimercapto-butanedioïque, diéthyl ester,
- l'acide 2,3-dimercapto-butanedioïque, dipropyl ester,
- l'acide 2,3-dimercapto-butanedioïque, bis(1-méthyléthyl)ester,
- l'acide 2,3-dimercapto-butanedioïque, dibutyl ester,
- l'acide 2,3-dimercapto-butanedioïque, bis(2-méthylpropyl)ester,
- l'acide 2,3-dimercapto-butanedioïque, bis(1,1-diméthyléthyl)ester,
- l'acide 2,3-dimercapto-butanedioïque, bis(1-méthylpropyl)ester, - l'acide 2,
- l'acide 2,3-dimercapto-butanedioïque, bis(3-méthylbutyl)ester,
- l'acide 2,3-dimercapto-butanedioïque, bis(1-ethylpropyl)ester,
- l'acide 2,3-dimercapto-butanedioïque, bis(2-méthoxyéthyl)ester,
- l'acide 2,3-dimercapto-butanedioïque, bis(2-éthoxyéthyl)ester,
- l'acide 2,4-dimercapto-5-méthylamino-5-oxo-pentanoïque,
- l'acide 2,4-dimercapto-5-éthylamino-5-oxo-pentanoïque,
- l'acide 2,4-dimercapto-5-propylamino-5-oxo-pentanoïque,
- l'acide 2,4-dimercapto-5-butylamino-5-oxo-pentanoïque,
- l'acide 2,4-dimercapto-5-[(1-méthyléthyl)amino]-5-oxo-pentanoïque,
- l'acide 2,4-dimercapto-5-[(2-méthylpropyl)amino]-5-oxo-pentanoïque,
- l'acide 2,4-dimercapto-5-[(3-méthylbutyl)amino]-5-oxo-pentanoïque,
- l'acide 2,4-dimercapto-5-[(2-hydroxyéthyl)amino]-5-oxo-pentanoïque,
- l'acide 2,4-dimercapto-5-[(2-hydroxypropyl)amino]-5-oxo-pentanoïque,
- l'acide 2,4-dimercapto-5-[(2-diméthylaminoéthyl)amino]-5-oxo-pentanoïque,
- l'acide 2,4-dimercapto-5-[(3-diméthylaminopropyl)amino]-5-oxo-pentanoïque,
- l'acide 2,4-dimercapto-pentanedioïque, monométhyl ester,
- l'acide 2,4-dimercapto-pentanedioïque, monoéthyl ester,
- l'acide 2,4-dimercapto-pentanedioïque, monopropyl ester,
- l'acide 2,4-dimercapto-pentanedioïque, mono(1-méthyléthyl)ester,
- le 2,4-dimercapto-pentanediamide,
- le 2,4-dimercapto-N,N'-diméthyl-pentanediamide,
- le 2,4-dimercapto-N,N'-diéthyl-pentanediamide,
- le 2,4-dimercapto-N,N'-bis (2-hydroxyéthyl)-pentanediamide,
- le 2,4-dimercapto-N,N'-bis(2-hydroxypropyl)-pentanediamide,
- l'acide 2,4-dimercapto-pentanedioïque, diméthyl ester,
- l'acide 2,4-dimercapto-pentanedioïque, diéthyl ester,
- l'acide 2,4-dimercapto-pentanedioïque, dipropyl ester,
- l'acide 2,4-dimercapto-pentanedioïque, dibutyl ester,
- l'acide 5-carbamoyl-2,5-dimercapto-valérique,
- l'acide 2,5-dimercapto-6-méthylamino-6-oxo-hexanoïque,
- l'acide 2,5-dimercapto-6-éthylamino-6-oxo-hexanoïque,
- l'acide 2,5-dimercapto-6-propylamino-6-oxo-hexanoïque,
- l'acide 2,5-dimercapto-6-[(2-hydroxyéthyl)amino]-6-oxo-hexanoïque,
- l'acide 2,5-dimercapto-6-[(2-hydroxypropyl)amino]-6-oxo-hexanoïque,
- l'acide 2,5-dimercapto-6-[(2-diméthylaminoéthyl)amino]-6-oxo-hexanoïque,
- l'acide 2,5-dimercapto-6-[(3-diméthylaminopropyl)amino]-6-oxo-hexanoïque,
- l'acide 2,5-dimercapto-hexanedioïque, monométhyl ester,
- l'acide 2,5-dimercapto-hexanedioïque, monoéthyl ester,
- l'acide 2,5-dimercapto-hexanedioïque, monopropyl ester,
- le 2,5-dimercapto -hexanediamide,
- le 2,5-dimercapto-N,N'-diméthyl-hexanediamide,
- le 2,5-dimercapto-N,N'-diéthyl-hexanediamide,
- le 2,5-dimercapto-N,N'-bis (2-hydroxyéthyl)-hexanediamide,
- le 2,5-dimercapto-N,N'-bis (2-hydroxypropyl)-hexanediamide,
- le 2,5-dimercapto-N,N,N',N'-tétraméthyl-hexanediamide,
- l'acide 2,5-dimercapto-hexanedioïque, diméthylester,
- l'acide 2,5-dimercapto-hexanedioïque, diéthylester,
- l'acide 2,5-dimercapto-hexanedioïque, dipropylester,
- l'acide 2,6-dimercapto-7-méthylamino-7-oxo-heptanedioïque,
- l'acide 2,6-dimercapto-7-éthylamino-7-oxo-heptanedioïque,
- l'acide 2,6-dimercapto-7-propylamino-7-oxo-heptanedioïque,
- l'acide 2,6-dimercapto-7-[(2-hydroxyéthyl)amino]-7-oxo-heptanedioïque,
- l'acide 2,6-dimercapto-7-[(2-hydroxypropyl)amino]-7-oxo-heptanedioïque,
- l'acide 2,6-dimercapto-7-[(2-diméthylaminoéthyl)amino]-7-oxo-heptanedioïque,
- l'acide 2,6-dimercapto-7-[(3-diméthylaminopropyl)amino]-7-oxo-heptanedioïque,
- l'acide 2,6-dimercapto-heptanedioïque, monométhyl ester,
- l'acide 2,6-dimercapto-heptanedioïque, monoéthyl ester,
- l'acide 2,6-dimercapto-heptanedioïque, monopropyl ester,
- le 2,6-dimercapto-heptanediamide,
- le 2,6-dimercapto-N,N'-diméthyl-heptanediamide,
- le 2,6-dimercapto-N,N'-bis(2-hydroxyéthyl)-heptanediamide,
- le 2,6-dimercapto-N,N'-bis(2-hydroxypropyl)-heptanediamide,
- l'acide 2,6-dimercapto-heptanedioïque, diméthylester,
- l'acide 2,6-dimercapto-heptanedioïque, diéthylester,
- l'acide 2,6-dimercapto-heptanedioïque, dipropylester,
- l'acide 2,7-dimercapto-8-méthylamino-8-oxo-octanedioïque,
- l'acide 2,7-dimercapto-8-éthylamino-8-oxo-octanedioïque,
- l'acide 2,7-dimercapto-8-propylamino-8-oxo-octanedioïque,
- l'acide 2,7-dimercapto-8-[(2-hydroxyéthyl)amino]-8-oxo-octanedioïque,
- l'acide 2,7-dimercapto-8-[(2-hydroxypropyl)amino]-8-oxo-octanedioïque,
- l'acide 2,7-dimercapto-8-[(2-diméthylaminoéthyl)amino]-8-oxo-octanedioïque,
- l'acide 2,7-dimercapto-8-[(3-diméthylaminopropyl)amino]-8-oxo-octanedioïque,
- l'acide 2,8-dimercapto-octanedioïque, monométhyl ester,
- l'acide 2,8-dimercapto-octanedioïque, monoéthyl ester,
- l'acide 2,8-dimercapto-octanedioïque, monopropyl ester,
- le 2,8-dimercapto-heptanediamide,
- le 2,8-dimercapto-N,N'-diméthyl-heptanediamide,
- le 2,8-dimercapto-N,N'-bis (2-hydroxyéthyl)-heptanediamide,
- le 2,8-dimercapto-N,N'-bis (2-hydroxypropyl)-heptanediamide,
- l'acide 2,8-dimercapto-heptanedioïque, diméthylester,
- l'acide 2,8-dimercapto-heptanedioïque, diéthylester,
- l'acide 2,8-dimercapto-heptanedioïque, dipropylester,
- l'acide 2,4-dimercapto-3-oxa-5-méthylamino-5-oxo-pentanoïque,
- l'acide 2,4-dimercapto-3-oxa-5-éthylamino-5-oxo-pentanoïque,
- l'acide 2,4-dimercapto-3-oxa-5-propylamino-5-oxo-pentanoïque,
- l'acide 2,4-dimercapto-3-oxa-5-butylamino-5-oxo-pentanoïque,
- l'acide 2,4-dimercapto-3-oxa-5-[(1-méthyléthyl)amino]-5-oxo-pentanoïque,
- l'acide 2,4-dimercapto-3-oxa-5-[(2-méthylpropyl)amino]-5-oxo-pentanoïque,
- l'acide 2,4-dimercapto-3-oxa-5-[(3-méthylbutyl)amino]-5-oxo-pentanoïque,
- l'acide 2,4-dimercapto-3-oxa-5-[(2-hydroxyéthyl)amino]-5-oxo-pentanoïque,
- l'acide 2,4-dimercapto-3-oxa-5-[(2-hydroxypropyl)amino]-5-oxo-pentanoïque,
- l'acide 2,4-dimercapto-3-oxa-5-[(2-diméthylaminoéthyl)amino]-5-oxo-pentanoïque,
- l'acide 2,4-dimercapto-3-oxa-5-[(3-diméthylaminopropyl)amino]-5-oxo-pentanoïque,
- l'acide 2,4-dimercapto-3-oxa-pentanedioïque, monométhyl ester,
- l'acide 2,4-dimercapto-3-oxa-pentanedioïque, monoéthyl ester,
- l'acide 2,4-dimercapto-3-oxa-pentanedioïque, monopropyl ester,
- l'acide 2,4-dimercapto-3-oxa-pentanedioïque, mono (1-méthyléthyl) ester,
- le 2,4-dimercapto-3-oxa-pentanediamide,
- le 2,4-dimercapto-3-oxa-N,N'-diméthyl-pentanediamide,
- le 2,4-dimercapto-3-oxa-N,N'-diéthyl-pentanediamide,
- le 2,4-dimercapto-3-oxa-N,N'-bis (2-hydroxyéthyl)-pentanediamide,
- le 2,4-dimercapto-3-oxa-N,N'-bis (2-hydroxypropyl)-pentanediamide,
- l'acide 2,4-dimercapto-3-oxa-pentanedioïque, diméthyl ester,
- l'acide 2,4-dimercapto-3-oxa-pentanedioïque, diéthyl ester,
- l'acide 2,4-dimercapto-3-oxa-pentanedioïque, dipropyl ester,
- l'acide 2,4-dimercapto-3-oxa-pentanedioïque, dibutyl ester,
- l'acide 2,6-dimercapto-4-thia-7-méthylamino-7-oxo-heptanedioïque,
- l'acide 2,6-dimercapto-4-thia-7-éthylamino-7-oxo-heptanedioïque,
- l'acide 2,6-dimercapto-4-thia-7-propylamino-7-oxo-heptanedioïque,
- l'acide 2,6-dimercapto-4-thia-7-[(2-hydroxyéthyl)amino]-7-oxo-heptanedioïque,
- l'acide 2,6-dimercapto-4-thia-7-[(2-hydroxypropyl)amino]-7-oxo-heptanedioïque,
- l'acide 2,6-dimercapto-4-thia-7-[(2-diméthylaminoéthyl)amino]-7-oxo
- heptanedioïque,
- l'acide 2,6-dimercapto-4-thia-7-[(3-diméthylaminopropyl)amino]-7-oxo-heptanedioïque,
- l'acide 2,6-dimercapto-4-thia-heptanedioïque, monométhyl ester,
- l'acide 2,6-dimercapto-4-thia-heptanedioïque, monoéthyl ester,
- l'acide 2,6-dimercapto-4-thia-heptanedioïque, monopropyl ester,
- le 2,6-dimercapto-4-thia-heptanediamide,
- le 2,6-dimercapto-4-thia-N,N'-diméthyl-heptanediamide,
- le 2,6-dimercapto-4-thia-N,N'-diéthyl-heptanediamide,
- le 2,6-dimercapto-4-thia-N,N'-bis (2-hydroxyéthyl)-heptanediamide,
- le 2,6-dimercapto-4-thia-N,N'-bis(2-hydroxypropyl)-heptanediamide,
- l'acide 2,6-dimercapto-4-thia-heptanedioïque, diméthylester,
- l'acide 2,6-dimercapto-4-thia-heptanedioïque, diéthylester,
- l'acide 2,6-dimercapto-4-thia-heptanedioïque, dipropylester,
- l'acide N-(carboxy-mercapto-méthyl)-2-mercapto-malonamique,
- l'acide [(carboxy-mercapto-méthyl)-méthyl-amino]-mercapto-acétique.

3. Composition selon la revendication 2 **caractérisée en ce que** le ou les dicarboxythiols de formule générale (1) et/ou de formule générale (11) sont choisis parmi :
- l'acide 2,3-dimercapto-4-méthylamino-4-oxo-butanoïque,
- l'acide 2,3-dimercapto-4-éthylamino-4-oxo-butanoïque,
- l'acide 2,3-dimercapto-4-propylamino-4-oxo-butanoïque,
- l'acide 2,3-dimercapto-4-butylamino-4-oxo-butanoïque,
- l'acide 2,3-dimercapto-4-[(2-hydroxyéthyl)amino]-4-oxo-butanoïque,
- l'acide 2,3-dimercapto-4-[(2-hydroxypropyl)amino]-4-oxo-butanoïque,
- l'acide 2,3-dimercapto-4-[(2-diméthylaminoéthyl)amino]-4-oxo-butanoïque,
- l'acide 2,3-dimercapto-4-[(3-diméthylaminopropyl)amino]-4-oxo-butanoïque,
- le 2,3dimercapto-butanediamide,
- le 2,3dimercapto-N,N'-diméthyl-butanediamide,
- le 2,3dimercapto-N,N'-bis(2-hydroxyéthyl)-butanediamide,
- le 2,3dimercapto-N,N'-bis(2-hydroxypropyl)-butanediamide,
- l'acide 2,4-dimercapto-5-méthylamino-5-oxo-pentanoïque,
- l'acide 2,4-dimercapto-5-éthylamino-5-oxo-pentanoïque,
- l'acide 2,4-dimercapto-5-propylamino-5-oxo-pentanoïque,
- l'acide 2,4-dimercapto-5-[(2-hydroxyéthyl)amino]-5-oxo-pentanoïque,
- l'acide 2,4-dimercapto-5-[(2-hydroxypropyl)amino]-5-oxo-pentanoïque,
- l'acide 2,4-dimercapto-5-[(2-diméthylaminoéthyl)amino]-5-oxo-pentanoïque,
- l'acide 2,4-dimercapto-5-[(3-diméthylaminopropyl)amino]-5-oxo-pentanoïque,
- le 2,4-dimercapto-pentanediamide,
- le 2,4-dimercapto-N,N'-diméthyl-pentanediamide,
- le 2,4-dimercapto-N,N'-bis(2-hydroxyéthyl)-pentanediamide,
- le 2,4-dimercapto-N,N'-bis(2-hydroxypropyl)-pentanediamide,
- l' acide 5-carbamoyl-2,5-dimercapto-valérique,
- l'acide 2,5-dimercapto-6-méthylamino-6-oxo-hexanoïque,
- l'acide 2,5-dimercapto-6-éthylamino-6-oxo-hexanoïque,
- l'acide 2,5-dimercapto-6-[(2-hydroxyéthyl)amino]-6-oxo-hexanoïque,
- l'acide 2,5-dimercapto-6-[(2-hydroxypropyl)amino]-6-oxo-hexanoïque,
- l'acide 2,5-dimercapto-6-[(2-diméthylaminoéthyl)amino]-6-oxo-hexanoïque,
- l'acide 2,5-dimercapto-6-[(3-diméthylaminopropyl)amino]-6-oxo-hexanoïque,
- le 2,5-dimercapto-hexanediamide,
- le 2,5-dimercapto-N,N'-diméthyl-hexanediamide,
- le 2,5-dimercapto-N,N'-diéthyl-hexanediamide,
- le 2,5-dimercapto-N,N'-bis(2-hydroxyéthyl)-hexanediamide,
- le 2,5-dimercapto-N,N'-bis(2-hydroxypropyl)-hexanediamide,
- l'acide 2,6-dimercapto-7-méthylamino-7-oxo-heptanedioïque,
- l'acide 2,6-dimercapto-7-éthylamino-7-oxo-heptanedioïque,
- l'acide 2,6-dimercapto-7-[(2-hydroxyéthyl)amino]-7-oxo-heptanedioïque,
- l'acide 2,6-dimercapto-7-[(2-hydroxypropyl)amino]-7-oxo-heptanedioïque,
- le 2,6-dimercapto-heptanediamide,
- le 2,6-dimercapto-N,N'-diméthyl-heptanediamide,
- le 2,6-dimercapto-N,N'-bis(2-hydroxyéthyl)-heptanediamide,
- le 2,6-dimercapto-N,N'-bis(2-hydroxypropyl)-heptanediamide,
- l'acide 2,7-dimercapto-8-méthylamino-8-oxo-octanedioïque,
- l'acide 2,7-dimercapto-8-éthylamino-8-oxo-octanedioïque,
- l'acide 2,7-dimercapto-8-[(2-hydroxyéthyl)amino]-8-oxo-octanedioïque,
- l'acide 2,7-dimercapto-8-[(2-hydroxypropyl)amino]-8-oxo-octanedioïque,
- le 2,8-dimercapto-heptanediamide,
- le 2,8-dimercapto-N,N'-diméthyl-heptanediamide,
- le 2,8-dimercapto-N,N'-bis(2-hydroxyéthyl)-heptanediamide,
- le 2,8-dimercapto-N,N'-bis(2-hydroxypropyl)-heptanediamide,
- l'acide 2,4-dimercapto-3-oxa-5-méthylamino-5-oxo-pentanoïque,
- l'acide 2,4-dimercapto-3-oxa-5-éthylamino-5-oxo-pentanoïque,
- l'acide 2,4-dimercapto-3-oxa-5-[(2-hydroxyéthyl)amino]-5-oxo-pentanoïque,
- l'acide 2,4-dimercapto-3-oxa-5-[(2-hydroxypropyl)amino]-5-oxo-pentanoïque,
- l'acide 2,4-dimercapto-3-oxa-5-[(2-diméthylaminoéthyl)amino]-5-oxo-pentanoïque,
- l'acide 2,4-dimercapto-3-oxa-5-[(3-diméthylaminopropyl)amino]-5-oxo-pentanoïque,
- le 2,4-dimercapto-3-oxa-pentanediamide,
- le 2,4-dimercapto-3-oxa-N,N'-diméthyl-pentanediamide,
- le 2,4-dimercapto-3-oxa-N,N'-bis(2-hydroxyéthyl)-pentanediamide,
- le 2,4-dimercapto-3-oxa-N,N'-bis(2-hydroxypropyl)-pentanediamide,
- l'acide 2,6-dimercapto-4-thia-7-méthylamino-7-oxo-heptanedioïque,
- l'acide 2,6-dimercapto-4-thia-7-éthylamino-7-oxo-heptanedioïque,
- l'acide 2,6-dimercapto-4-thia-7-[(2-hydroxyéthyl)amino]-7-oxo-heptanedioïque,
- l'acide 2,6-dimercapto-4-thia-7-[(2-hydroxypropyl)amino]-7-oxo-heptanedioïque,
- le 2,6-dimercapto-4-thia-heptanediamide,
- le 2,6-dimercapto-4-thia-N,N'-diméthyl-heptanediamide,
- le 2,6-dimercapto-4-thia-N,N'-diéthyl-heptanediamide,
- le 2,6-dimercapto-4-thia-N,N'-bis(2-hydroxyéthyl)-heptanediamide,
- le 2,6-dimercapto-4-thia-N,N'-bis(2-hydroxypropyl)-heptanediamide.

4. Composition selon l'une quelconque des revendications précédentes **caractérisée en ce que** le ou les dicarboxydithiols de formule générale (1) et/ou (11), et/ou leurs sels organiques et minéraux, sont présents à une teneur comprise entre 0,05 et 30%, de préférence entre 1 et 20%, en poids par rapport au poids total de la composition.

5. Composition selon l'une quelconque des revendications précédentes **caractérisée en ce que** le pH est compris entre 4 et 11, de préférence entre 6 et 10.

6. Composition selon la revendication 5 **caractérisé en ce que** le pH est obtenu à l'aide d'un agent alcalin choisi parmi l'ammoniaque, la monoéthanolamine, la diéthanolamine, la propanediamine-1,3, un carbonate ou bicarbonate alcalin ou d'ammonium, un carbonate organique, de préférence le carbonate de guanidine, et un hydroxyde alcalin, ou bien à l'aide d'un agent acidifiant choisi parmi l'acide chlorhydrique, l'acide acétique, l'acide lactique, l'acide oxalique ou l'acide borique, ou bien à l'aide d'un tampon borate, phosphate ou TRIS.

7. Composition selon l'une quelconque des revendications précédentes **caractérisée en ce qu'**elle comprend en outre un ou plusieurs autres agents réducteurs choisis parmi l'acide thioglycolique, l'acide thiolactique, le monothioglycolate de glycérol, la cystéamine, la N-acétyl-cystéamine, la N-propionyl-cystéamine, la cystéine, la N-acétyl-cystéine, l'acide thiomalique, la panthétéine, l'acide 2,3-dimercaptosuccinique, les sulfites ou bisulfites d'un métal alcalin ou alcalino-terreux, les N-(mercaptoaikyi)-ω-hydroxyalkylamides, les N-mono ou N,N-dialkylmercapto-4-butyramides, les aminomercapto-alkylamides, les dérivés des acides N-(mercaptoalkyl)succinamiques et des N-(mercaptoalkyl)succinimides, les alkylamino mercaptoalkylamides, le mélange azéotrope de thioglyconate de 2-hydroxypropyle et de thioglycolate de (2-hydroxy-1-méthyl)éthyle, les mercaptoalkylaminoamides, les N-mercapto-alkylalcanediamides et les dérivés d'acide formamidine sulfinique.

8. Composition selon l'une quelconque des revendications précédentes **caractérisée en ce qu'**elle comprend au moins un agent tensioactif choisi parmi les alkyl sulfates, les alkyl benzènesulfates, les alkyl éthersulfates, les alkyl sulfonates, les sels d'ammonium quaternaire, les alkyl bétaïnes, les alkyl phénols oxyéthylénés, les alcanolamides d'acides gras, les esters d'acides gras oxyéthylénés, les hydroxypropyléthers.

9. Composition selon la revendication 8 **caractérisée en ce que** le ou les agents tensioactifs représentent au plus 30% en poids, de préférence entre 0,5 et 10% en poids, du poids total de la composition.

10. Composition selon l'une quelconque des revendications précédentes **caractérisée en ce qu'**elle comprend un véhicule choisi parmi l'eau et une solution hydroalcoolique d'un alcool inférieur en C₁-C₆, de préférence l'éthanol, l'isopropanol ou le butanol.

11. Composition selon l'une quelconque des revendications précédentes **caractérisée en ce qu'**elle se présente sous forme aqueuse, de préférence sous la forme d'une lotion, épaissie ou non, d'une crème, épaissie ou non, ou d'un gel.

12. Procédé de déformation permanente des cheveux comprenant :
- une étape d'application sur les cheveux d'une composition réductrice telle que définie dans l'une quelconque des revendications 1 à 11, pour réduire les liaisons disulfures de la kératine,
- une étape de fixation par oxydation, pour reformer lesdites liaisons, par application d'une composition oxydante sur les cheveux ou par mise en contact des cheveux avec l'oxygène de l'air.

13. Procédé selon la revendication 12 **caractérisé en ce que** on laisse agir la composition réductrice pendant 5 à 60 minutes, de préférence pendant 15 à 45 minutes.

14. Procédé selon la revendication 13 **caractérisé en ce que** la composition oxydante comprend au moins un agent oxydant choisi parmi l'eau oxygénée, les bromates alcalins, les persels, les polythionates et un mélange de bromate alcalin et de persel.

15. Procédé selon l'une quelconque des revendications 12 à 14 **caractérisé en ce qu'**il comprend, entre l'étape d'application sur les cheveux de la composition réductrice et l'étape de fixation, une étape de chauffage des cheveux avec un fer chauffant à une température comprise entre 60 et 220°C, de préférence entre 120 et 200°C.

16. Kit pour la déformation permanente des cheveux comprenant dans un premier compartiment une composition réductrice telle que définie dans l'une quelconque des revendications 1 à 11, et dans un second compartiment une composition oxydante.

17. Dicarboxydithiol **caractérisé en ce qu'**il répond à la formule générale (III) ou à la formule générale (IV) suivantes : 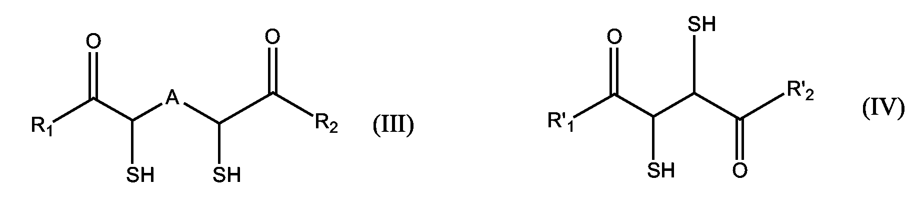 dans lesquelles :
A, R₁, R₂, R'₁ et R'₂ sont tels que définis dans la revendication 1, à l'exclusion des composés dans lesquels :
(i) R'₁ représente OH et R'₂ représente OR₃, R₃ étant un radical alkyle linéaire ou ramifié en C₁-C₅, ou bien R'₂ représente NR₄R₅, R₄ étant un atome d'hydrogène et R₅ un radical alkyle ramifié en C₃-C₅ ;
(ii) R'₁ et R'₂ représentent un radical amino ;
(iii) R'₁ et R'₂ représentent le radical OR3 dans lequel R3 désigne un radical alkyle linéaire ou ramifié en C₁-C₅ ainsi que les radicaux 2-méthoxyéthyle et 2-éthoxyéthyle ;
(iv) A représente CH₂ et R₁ et R₂ représentent un radical amino ;
(v) A représente CH₂ et R₁ et R₂ représentent le radical OR₃ dans lequel R₃ désigne un radical butyle ;
(vi) A représente (CH₂)₂, R₁ représente OH et R₂ représente NR₄R₅, R₄ étant un atome d'hydrogène et R₅ un atome d'hydrogène ;
(vii) A représente (CH₂)₂ et R₁ et R₂ représentent un radical diméthylamino ;
(viii) A représente (CH₂)₂ et R₁ et R₂ représentent le radical OR₃ dans lequel R₃ désigne un radical méthyle ;
ou un sel organique ou minéral dudit composé de formule (III) ou (IV).

18. Dicarboxydithiol selon la revendication 17 **caractérisé en ce qu'**il est choisi parmi les composés suivants :
- l'acide 2,3-dimercapto-4-méthylamino-4-oxo-butanoïque,
- l'acide 2,3-dimercapto-4-éthylamino-4-oxo-butanoïque,
- l'acide 2,3-dimercapto-4-propylamino-4-oxo-butanoïque,
- l'acide 2,3-dimercapto-4-butylamino-4-oxo-butanoïque,
- l'acide 2,3-dimercapto-4-[(2-hydroxyéthyl)amino]-4-oxo-butanoïque,
- l'acide 2,3-dimercapto-4-[(2-hydroxypropyl)amino]-4-oxo-butanoïque,
- l'acide 2,3-dimercapto-4-[(2-diméthylaminoéthyl)amino]-4-oxo-butanoïque,
- l'acide 2,3-dimercapto-4-[(3-diméthylaminopropyl)amino]-4-oxo-butanoïque,
- le 2,3 dimercapto-N,N'-diméthyl-butanediamide,
- le 2,3 dimercapto-N,N'-diéthyl-butanediamide,
- le 2,3 dimercapto-N,N'-bis(2-hydroxyéthyl)-butanediamide,
- le 2,3 dimercapto-N,N'-bis(2-hydroxypropyl)-butanediamide,
- l'acide 2,4-dimercapto-5-méthylamino-5-oxo-pentanoïque,
- l'acide 2,4-dimercapto-5-éthylamino-5-oxo-pentanoïque,
- l'acide 2,4-dimercapto-5-propylamino-5-oxo-pentanoïque,
- l'acide 2,4-dimercapto-5-butylamino-5-oxo-pentanoïque,
- l'acide 2,4-dimercapto-5-[(1-méthyléthyl)amino]-5-oxo-pentanoïque,
- l'acide 2,4-dimercapto-5-[(2-méthylpropyl)amino]-5-oxo-pentanoïque,
- l'acide 2,4-dimercapto-5-[(3-méthylbutyl)amino]-5-oxo-pentanoïque,
- l'acide 2,4-dimercapto-5-[(2-hydroxyéthyl)amino]-5-oxo-pentanoïque,
- l'acide 2,4-dimercapto-5-[(2-hydroxypropyl)amino]-5-oxo-pentanoïque,
- l'acide 2,4-dimercapto-5-[(2-diméthylaminoéthyl)amino]-5-oxo-pentanoïque,
- l'acide 2,4-dimercapto-5-[(3-diméthylaminopropyl)amino]-5-oxo-pentanoïque,
- l'acide 2,4-dimercapto-pentanedioïque, monométhyl ester,
- l'acide 2,4-dimercapto-pentanedioïque, monoéthyl ester,
- l'acide 2,4-dimercapto-pentanedioïque, monopropyl ester,
- l'acide 2,4-dimercapto-pentanedioïque,mono(1-méthyléthyl) ester,
- le 2,4-dimercapto-N,N'-diméthyl-pentanediamide,
- le 2,4-dimercapto-N,N'-diéthyl-pentanediamide,
- le 2,4-dimercapto-N,N'-bis(2-hydroxyéthyl)-pentanediamide,
- le 2,4-dimercapto-N,N'-bis(2-hydroxypropyl)-pentanediamide,
- l'acide 2,4-dimercapto-pentanedioïque, diméthyl ester,
- l'acide 2,4-dimercapto-pentanedioïque, diéthyl ester,
- l'acide 2,4-dimercapto-pentanedioïque, dipropyl ester,
- l'acide 2,5-dimercapto-6-méthylamino-6-oxo-hexanoïque,
- l'acide 2,5-dimercapto-6-éthylamino-6-oxo-hexanoïque,
- l'acide 2,5-dimercapto-6-propylamino-6-oxo-hexanoïque,
- l'acide 2,5-dimercapto-6-[(2-hydroxyéthyl)amino]-6-oxo-hexanoïque,
- l'acide 2,5-dimercapto-6-[(2-hydroxypropyl)amino]-6-oxo-hexanoïque,
- l'acide 2,5-dimercapto-6-[(2-diméthylaminoéthyl)amino]-6-oxo-hexanoïque,
- l'acide 2,5-dimercapto-6-[(3-diméthylaminopropyl)amino]-6-oxo-hexanoïque
- l'acide 2,5-dimercapto-hexanedioïque, monométhyl ester,
- l'acide 2,5-dimercapto-hexanedioïque, monoéthyl ester,
- l'acide 2,5-dimercapto-hexanedioïque, monopropyl ester,
- le 2,5-dimercapto-hexanediamide,
- le 2,5-dimercapto-N,N'-diméthyl-hexanediamide,
- le 2,5-dimercapto-N,N'-diéthyl-hexanediamide,
- le 2,5-dimercapto-N,N'-bis(2-hydroxyéthyl)-hexanediamide,
- le 2,5-dimercapto-N,N'-bis(2-hydroxypropyl)-hexanediamide,
- l'acide 2,5-dimercapto-hexanedioïque, diéthylester,
- l'acide 2,5-dimercapto-hexanedioïque, dipropylester,
- l'acide 2,6-dimercapto-7-méthylamino-7-oxo-heptanedioïque,
- l'acide 2,6-dimercapto-7-éthylamino-7-oxo-heptanedioïque,
- l'acide 2,6-dimercapto-7-propylamino-7-oxo-heptanedioïque,
- l'acide 2,6-dimercapto-7-[(2-hydroxyéthyl)amino]-7-oxo-heptanedioïque,
- l'acide 2,6-dimercapto-7-[(2-hydroxypropyl)amino]-7-oxo-heptanedioïque,
- l'acide 2,6-dimercapto-7-[(2-diméthylaminoéthyl)amino]-7-oxo-heptanedioïque,
- l'acide 2,6-dimercapto-7-[(3-diméthylaminopropyl)amino]-7-oxo-heptanedioïque,
- l'acide 2,6-dimercapto-heptanedioïque, monométhyl ester,
- l'acide 2,6-dimercapto-heptanedioïque, monoéthyl ester,
- l'acide 2,6-dimercapto-heptanedioïque, monopropyl ester,
- le 2,6-dimercapto-heptanediamide,
- le 2,6-dimercapto-N,N'-diméthyl-heptanediamide,
- le 2,6-dimercapto-N,N'-bis(2-hydroxyéthyl)-heptanediamide,
- le 2,6-dimercapto-N,N'-bis(2-hydroxypropyl)-heptanediamide,
- l'acide 2,6-dimercapto-heptanedioïque, diméthylester,
- l'acide 2,6-dimercapto-heptanedioïque, diéthylester,
- l'acide 2,6-dimercapto-heptanedioïque, dipropylester,
- l'acide 2,7-dimercapto-8-méthylamino-8-oxo-octanedioïque,
- l'acide 2,7-dimercapto-8-éthylamino-8-oxo-octanedioïque,
- l'acide 2,7-dimercapto-8-propylamino-8-oxo-octanedioïque,
- l'acide 2,7-dimercapto-8-[(2-hydroxyéthyl)amino]-8-oxo-octanedioïque,
- l'acide 2,7-dimercapto-8-[(2-hydroxypropyl)amino]-8-oxo-octanedioïque,
- l'acide 2,7-dimercapto-8-[(2-diméthylaminoéthyl)amino]-8-oxo-octanedioïque,
- l'acide 2,7-dimercapto-8-[(3-diméthylaminopropyl)amino]-8-oxo-octanedioïque,
- l'acide 2,8-dimercapto-octanedioïque, monométhyl ester,
- l'acide 2,8-dimercapto-octanedioïque, monoéthyl ester,
- l'acide 2,8-dimercapto-octanedioïque, monopropyl ester,
- le 2,8-dimercapto-heptanediamide,
- le 2,8-dimercapto-N,N'-diméthyl-heptanediamide,
- le 2,8-dimercapto-N,N'-bis (2-hydroxyéthyl)-heptanediamide,
- le 2,8-dimercapto-N,N'-bis (2-hydroxypropyl)-heptanediamide,
- l'acide 2,8-dimercapto-heptanedioïque, diméthylester,
- l'acide 2,8-dimercapto-heptanedioïque, diéthylester,
- l'acide 2,8-dimercapto-heptanedioïque, dipropylester,
- l'acide 2,4-dimercapto-3-oxa-5-méthylamino-5-oxo-pentanoïque,
- l'acide 2,4-dimercapto-3-oxa-5-éthylamino-5-oxo-pentanoïque,
- l'acide 2,4-dimercapto-3-oxa-5-propylamino-5-oxo-pentanoïque,
- l'acide 2,4-dimercapto-3-oxa-5-butylamino-5-oxo-pentanoïque,
- l'acide 2,4-dimercapto-3-oxa-5-[(1-méthyléthyl)amino]-5-oxo-pentanoïque,
- l'acide 2,4-dimercapto-3-oxa-5-[(2-méthylpropyl)amino]-5-oxo-pentanoïque,
- l'acide 2,4-dimercapto-3-oxa-5-[(3-méthylbutyl)amino]-5-oxo-pentanoïque,
- l'acide 2,4-dimercapto-3-oxa-5-[(2-hydroxyéthyl)amino]-5-oxo-pentanoïque,
- l'acide 2,4-dimercapto-3-oxa-5-[(2-hydroxypropyl)amino]-5-oxo-pentanoïque,
- l'acide 2,4-dimercapto-3-oxa-5-[(2-diméthylaminoéthyl)amino]-5-oxo-pentanoïque,
- l'acide 2,4-dimercapto-3-oxa-5-[(3-diméthylaminopropyl)amino]-5-oxo-pentanoïque,
- l'acide 2,4-dimercapto-3-oxa-pentanedioïque, monométhyl ester,
- l'acide 2,4-dimercapto-3-oxa-pentanedioïque, monoéthyl ester,
- l'acide 2,4-dimercapto-3-oxa-pentanedioïque, monopropyl ester,
- l'acide 2,4-dimercapto-3-oxa-pentanedioïque,mono(1-méthyléthyl) ester,
- le 2,4-dimercapto-3-oxa-pentanediamide,
- le 2,4-dimercapto-3-oxa-N,N'-diméthyl-pentanediamide,
- le 2,4-dimercapto-3-oxa-N,N'-diéthyl-pentanediamide,
- le 2,4-dimercapto-3-oxa-N,N'-bis (2-hydroxyéthyl)-pentanediamide,
- le 2,4-dimercapto-3-oxa- N,N'-bis (2-hydroxypropyl)-pentanediamide,
- l'acide 2,4-dimercapto-3-oxa-pentanedioïque, diméthyl ester,
- l'acide 2,4-dimercapto-3-oxa-pentanedioïque, diéthyl ester,
- l'acide 2,4-dimercapto-3-oxa-pentanedioïque, dipropyl ester,
- l'acide 2,4-dimercapto-3-oxa-pentanedioïque, dibutyl ester,
- l'acide 2,6-dimercapto-4-thia-7-méthylamino-7-oxo-heptanedioïque,
- l'acide 2,6-dimercapto-4-thia-7-éthylamino-7-oxo-heptanedioïque,
- l'acide 2,6-dimercapto-4-thia-7-propylamino-7-oxo-heptanedioïque,
- l'acide 2,6-dimercapto-4-thia-7-[(2-hydroxyéthyl)amino]-7-oxo-heptanedioïque,
- l'acide 2,6-dimercapto-4-thia-7-[(2-hydroxypropyl)amino]-7-oxo-heptanedioïque,
- l'acide 2,6-dimercapto-4-thia-7-[(2-diméthylaminoéthyl)amino]-7-oxo-heptanedioïque,
- l'acide 2,6-dimercapto-4-thia-7-[(3-diméthylaminopropyl)amino]-7-oxo - heptanedioïque,
- l'acide 2,6-dimercapto-4-thia-heptanedioïque, monométhyl ester,
- l'acide 2,6-dimercapto-4-thia-heptanedioïque, monoéthyl ester,
- l'acide 2,6-dimercapto-4-thia-heptanedioïque, monopropyl ester,
- le 2,6-dimercapto-4-thia-heptanediamide,
- le 2,6-dimercapto-4-thia-N,N'-diméthyl-heptanediamide,
- le 2,6-dimercapto-4-thia-N,N'-diéthyl-heptanediamide,
- le 2,6-dimercapto-4-thia-N,N'-bis(2-hydroxyéthyl)-heptanediamide,
- le 2,6-dimercapto-4-thia-N,N'-bis(2-hydroxypropyl)-heptanediamide,
- l'acide 2,6-dimercapto-4-thia-heptanedioïque, diméthylester,
- l'acide 2,6-dimercapto-4-thia-heptanedioïque, diéthylester,
- l'acide 2,6-dimercapto-4-thia-heptanedioïque, dipropylester,
- l'acide N-(carboxy-mercapto-méthyl)-2-mercapto-malonamique,
- l'acide [(carboxy-mercapto-méthyl)-méthyl-amino]-mercapto-acétique.

19. Dicarboxydithiol selon la revendication 18 **caractérisé en ce qu'**il est choisi parmi les composés suivants :
- l'acide 2,3-dimercapto-4-méthylamino-4-oxo-butanoïque,
- l'acide 2,3-dimercapto-4-éthylamino-4-oxo-butanoïque,
- l'acide 2,3-dimercapto-4-propylamino-4-oxo-butanoïque,
- l'acide 2,3-dimercapto-4-butylamino-4-oxo-butanoïque,
- l'acide 2,3-dimercapto-4-[(2-hydroxyéthyl)amino]-4-oxo-butanoïque,
- l'acide 2,3-dimercapto-4-[(2-hydroxypropyl)amino]-4-oxo-butanoïque,
- l'acide 2,3-dimercapto-4-[(2-diméthylaminoéthyl)amino]-4-oxo-butanoïque,
- l'acide 2,3-dimercapto-4-[(3-diméthylaminopropyl)amino]-4-oxo-butanoïque,
- le 2,3dimercapto-N,N'-diméthyl-butanediamide,
- le 2,3dimercapto-N,N'-bis(2-hydroxyéthyl)-butanediamide,
- le 2,3dimercapto-N,N'-bis(2-hydroxypropyl)-butanediamide,
- l'acide 2,4-dimercapto-5-méthylamino-5-oxo-pentanoïque,
- l'acide 2,4-dimercapto-5-éthylamino-5-oxo-pentanoïque,
- l'acide 2,4-dimercapto-5-propylamino-5-oxo-pentanoïque,
- l'acide 2,4-dimercapto-5-[(2-hydroxyéthyl)amino]-5-oxo-pentanoïque,
- l'acide 2,4-dimercapto-5-[(2-hydroxypropyl)amino]-5-oxo-pentanoïque,
- l'acide 2,4-dimercapto-5-[(2-diméthylaminoéthyl)amino]-5-oxo-pentanoïque,
- l'acide 2,4-dimercapto-5-[(3-diméthylaminopropyl)amino]-5-oxo-pentanoïque,
- le 2,4-dimercapto-N,N'-diméthyl-pentanediamide,
- le 2,4-dimercapto-N,N'-bis(2-hydroxyéthyl)-pentanediamide,
- le 2,4-dimercapto-N,N'-bis(2-hydroxypropyl)-pentanediamide,
- l'acide 2,5-dimercapto-6-méthylamino-6-oxo-hexanoïque,
- l'acide 2,5-dimercapto-6-éthylamino-6-oxo-hexanoïque,
- l'acide 2,5-dimercapto-6-[(2-hydroxyéthyl)amino]-6-oxo-hexanoïque,
- l'acide 2,5-dimercapto-6-[(2-hydroxypropyl)amino]-6-oxo-hexanoïque,
- l'acide 2,5-dimercapto-6-[(2-diméthylaminoéthyl)amino]-6-oxo-hexanoïque,
- l'acide 2,5-dimercapto-6-[(3-diméthylaminopropyl)amino]-6-oxo-hexanoïque,
- le 2,5-dimercapto-hexanediamide,
- le 2,5-dimercapto-N,N'-diméthyl-hexanediamide,
- le 2,5-dimercapto-N,N'-diéthyl-hexanediamide,
- le 2,5-dimercapto-N,N'-bis(2-hydroxyéthyl)-hexanediamide,
- le 2,5-dimercapto-N,N'-bis (2-hydroxypropyl)-hexanediamide,
- l'acide 2,6-dimercapto-7-méthylamino-7-oxo-heptanedioïque,
- l'acide 2,6-dimercapto-7-éthylamino-7-oxo-heptanedioïque,
- l'acide 2,6-dimercapto-7-[(2-hydroxyéthyl)amino]-7-oxo-heptanedioïque,
- l'acide 2,6-dimercapto-7-[(2-hydroxypropyl)amino]-7-oxo-heptanedioïque,
- le 2,6-dimercapto-heptanediamide,
- le 2,6-dimercapto-N,N'-diméthyl-heptanediamide,
- le 2,6-dimercapto-N,N'-bis(2-hydroxyéthyl)-heptanediamide,
- le 2,6-dimercapto-N,N'-bis(2-hydroxypropyl)-heptanediamide,
- l'acide 2,7-dimercapto-8-méthylamino-8-oxo-octanedioïque,
- l'acide 2,7-dimercapto-8-éthylamino-8-oxo-octanedioïque,
- l'acide 2,7-dimercapto-8-[(2-hydroxyéthyl)amino]-8-oxo-octanedioïque,
- l'acide 2,7-dimercapto-8-[(2-hydroxypropyl)amino]-8-oxo-octanedioïque,
- le 2,8-dimercapto-heptanediamide,
- le 2,8-dimercapto-N,N'-diméthyl-heptanediamide,
- le 2,8-dimercapto-N,N'-bis (2-hydroxyéthyl)-heptanediamide,
- le 2,8-dimercapto-N,N'-bis (2-hydroxypropyl)-heptanediamide,
- l'acide 2,4-dimercapto-3-oxa-5-méthylamino-5-oxo-pentanoïque,
- l'acide 2,4-dimercapto-3-oxa-5-éthylamino-5-oxo-pentanoïque,
- l'acide 2,4-dimercapto-3-oxa-5-[(2-hydroxyéthyl)amino]-5-oxo-pentanoïque,
- l'acide 2,4-dimercapto-3-oxa-5-[(2-hydroxypropyl)amino]-5-oxo-pentanoïque,
- l'acide 2,4-dimercapto-3-oxa-5-[(2-hydroxypropyl)amino]-5-oxo-pentanoïque,
- l'acide 2,4-dimercapto-3-oxa-5-[(3-diméthylaminopropyl)amino]-5-oxo-pentanoïque,
- le 2,4-dimercapto-3-oxa-pentanediamide,
- le 2,4-dimercapto-3-oxa-N,N'-diméthyl-pentanediamide,
- le 2,4-dimercapto-3-oxa-N,N'-bis(2-hydroxyéthyl)-pentanediamide,
- le 2,4-dimercapto-3-oxa-N,N'-bis(2-hydroxypropyl)-pentanediamide,
- l'acide 2,6-dimercapto-4-thia-7-méthylamino-7-oxo-heptanedioïque,
- l'acide 2,6-dimercapto-4-thia-7-éthylamino-7-oxo-heptanedioïque,
- l'acide 2,6-dimercapto-4-thia-7-[(2-hydroxyéthyl)amino]-7-oxo-heptanedioïque,
- l'acide 2,6-dimercapto-4-thia-7-[(2-hydroxypropyl)amino]-7-oxo- heptanedioïque,
- le 2,6-dimercapto-4-thia-heptanediamide,
- le 2,6-dimercapto-4-thia-N,N'-diméthyl-heptanediamide,
- le 2,6-dimercapto-4-thia-N,N'-diéthyl-heptanediamide,
- le 2,6-dimercapto-4-thia-N,N'-bis(2-hydroxyéthyl)-heptanediamide,
- le 2,6-dimercapto-4-thia-N,N'-bis(2-hydroxypropyl)-heptanediamide.
